(19) Europäisches Patentamt
European Patent Office
Office européen des brevets

(11) **EP 3 279 322 B1**

(12) **EUROPEAN PATENT SPECIFICATION**

(45) Date of publication and mention
of the grant of the patent:
**01.01.2020 Bulletin 2020/01**

(51) Int Cl.:
***C12N 15/09*** (2006.01)        ***C12Q 1/68*** (2018.01)

(21) Application number: **16773013.4**

(86) International application number:
**PCT/JP2016/060480**

(22) Date of filing: **30.03.2016**

(87) International publication number:
**WO 2016/159132 (06.10.2016 Gazette 2016/40)**

(54) **METHOD FOR DESIGNING PRIMERS TO BE SUBJECTED TO POLYMERASE CHAIN REACTION, AND PRIMER SET**

VERFAHREN ZUM ENTWURF VON PRIMERN, DIE EINER POLYMERASE-KETTENREAKTION UNTERZOGEN WERDEN SOLLEN, UND PRIMER-SET

PROCÉDÉ POUR CONCEVOIR DES AMORCES À SOUMETTRE À UNE RÉACTION EN CHAÎNE PAR POLYMÉRASE, ET ENSEMBLE D'AMORCES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2015 JP 2015074299**

(43) Date of publication of application:
**07.02.2018 Bulletin 2018/06**

(73) Proprietor: **FUJIFILM Corporation
Tokyo 106-8620 (JP)**

(72) Inventor: **TSUJIMOTO Takayuki
Ashigara-kami-gun
Kanagawa 258-8538 (JP)**

(74) Representative: **Klunker IP
Patentanwälte PartG mbB
Destouchesstraße 68
80796 München (DE)**

(56) References cited:
**WO-A1-2008/004691        WO-A1-2010/080691
JP-A- 2009 533 030        JP-A- 2011 062 085
US-A1- 2009 305 288        US-A1- 2013 123 120**

• **POE L. BRIAN ET AL.: 'Warfarin Genotyping in a Single PCR Reaction for Microchip Electrophoresis' CLINICAL CHEMISTRY vol. 58, no. 4, 2012, pages 725 - 731, XP055315428**

**Description**

**BACKGROUND OF THE INVENTION**

1. Field of the Invention

The present invention relates to a method for designing a primer used for a polymerase chain reaction. A primer set is also described.

2. Description of the Related Art

In recent years, it has become easier to guarantee the quality and the amount of base sequence data due to the spread of next generation sequencing technology, and therefore, genetic analysis has become easier. Many technical difficulties in whole genome analysis are being resolved due to introduction of next generation sequencing (NGS) technology. However, the total base length of the genome is generally enormous, for example, greater than or equal to 3 billion base pairs in a case of the human genome. Even with NGS technology, it requires considerable cost and time to perform whole genome analysis.

On the other hand, it cannot be said that the whole genome analysis is optimal as means for achieving the purpose of detecting gene abnormality. This is because it is sufficient to analyze only a gene region relating to the gene abnormality. The gene region relating to the gene abnormality includes both a coding region and a non-coding region. For this reason, a polymerase chain reaction (PCR) method has been spreading as a technique of efficiently performing genetic analysis with accuracy, by amplifying only a necessary specific gene region and performing limited reading on a base sequence thereof. Particularly, a method for selectively amplifying a plurality of gene regions by simultaneously supplying a plurality of types of primers to a certain PCR reaction system is called multiplex PCR.

However, in general, it is impossible to set the number of regions simultaneously amplified through a multiplex PCR to be excessively large. As one of the factors, there is a problem in that unnecessary amplification products called primer dimers are generated through a reaction between primers, and therefore, it is impossible to efficiently amplify an objective gene region. This problem is remarkable in a case of, for example, single cell analysis, where the amount of deoxyribonucleic acid (DNA) which becomes a template for a PCR reaction is extremely small.

Means for subjecting a large number of regions to PCR by dividing a base sequence of primers into a constant region and a variable region, arranging identical base sequences in the constant region, and limiting the number of bases in the variable region to be only two, which may not become complementary to each other, out of cytosine (C), thymidine (T), guanine (G), and adenine (A) is disclosed, for example, in WO2004/081225A as means for suppressing the formation of primer dimers. In addition, it is disclosed in WO2008/004691A that the possibility that different target primers may form a primer dimer through multiplex PCR, by calculating a score (local alignment score at the 3' terminal) showing complementarity at the 3' terminal between primers regarding all combinations of primers and selecting a combination of primers of which the complementarity to each other is low.

US 2013/123 120 A1 discloses a method of selecting test primers from a library of candidate primers, the method comprising calculating on a computer an undesirability score for most or all of the possible combinations of two candidate primers from the library, wherein each undesirability score is based at least in part on the likelihood of dimer formation between the two candidate primers; and removing from the library of candidate primers the candidate primer that is part of the greatest number of combinations of two candidate primers with an undesirability score above a first minimum threshold, thereby selecting a library of test primers.

JP 2009 533 030 A describes a primer set used in real-time multiplex amplification, and discloses that HPV genome fragments are determined through global alignment.

Poe, Brian L. et al.: "Warfarin Genotyping in a Single PCR Reaction for Microchip Electrophoresis", Clinical Chemistry, vol. 58, no. 4, 2012, pages 725-731, discloses that a primer for genotyping in a single PCR reaction was designed through local alignment.

**SUMMARY OF THE INVENTION**

As a result of studies of the present inventors, an object of the means disclosed in WO2004/081225A is to provide amplification means which is not deviated with respect to the whole genome region by providing a universal primer, and therefore, only a region that is interposed between base sequences which include specific variable regions can be set as a target, and it is impossible to efficiently select only a plurality of the specific regions. In addition, it is important to consider a primer dimer which is formed through annealing of only primer ends in a PCR reaction with respect to a trace amount of template DNA, such as a multiplex PCR from a single cell. However, it is not considered in the means disclosed in WO2008/004691A. The result shows that, in the means disclosed in WO2004/081225A and WO2008/004691A, it has been impossible to efficiently perform selective amplification of only a plurality of regions on the genome at a level required in these days.

Therefore, an object of the present invention is to provide a method for designing a primer used for polymerase chain reaction which can selectively amplify an objective gene region efficiently.

The present inventors have conducted extensive studies to solve the above-described problems. As a result, they have found that a primer set can be obtained which is used for a polymerase chain reaction and can selectively amplify an objective gene region efficiently in a case where primers selected in both a first stage and a second stage are employed by performing: first stage selection based on a local alignment score obtained by evaluating formability of a primer dimer and obtaining a local alignment score through performing pairwise local alignment on a base sequence of a primer candidate under the condition that a partial sequence to be subjected to comparison includes the 3' terminal of a base sequence of a primer; and a second stage selection based on a global alignment score obtained by performing pairwise global alignment on a base sequence which is of an up to three base length and includes the 3' terminal of the base sequence of the primer candidate, and have completed the present invention.

That is, the present invention is the following (1) and (2).

(1) A method for designing a primer used for a polymerase chain reaction, the method comprising: a target region selection step of selecting a target region to be amplified through the polymerase chain reaction, from regions on a genome; a primer candidate base sequence generation step of generating at least one base sequence of a primer candidate for amplifying the target region based on each base sequence in vicinity regions at both ends of the target region on the genome; a local alignment step of obtaining a local alignment score by performing pairwise local alignment on the base sequence of the primer candidate under a condition that a partial sequence to be subjected to comparison includes the 3' terminal of the base sequence of the primer candidate; a first stage selection step of performing first stage selection of the base sequence of the primer candidate based on the local alignment score obtained in the local alignment step; a global alignment step of obtaining a global alignment score by performing pairwise global alignment on a base sequence which is of an up to three base length and includes the 3' terminal of the base sequence of the primer candidate; a second stage selection step of performing second stage selection of a base sequence of the primer candidate based on the global alignment score obtained in the global alignment step; and a primer employment step of employing the base sequence of the primer candidate which has been selected in both of the first stage selection step and the second stage selection step as a base sequence of a primer for amplifying the target region, in which both steps of the local alignment step and the first stage selection step are performed before or after both steps of the global alignment step and the second stage selection step, or performed in parallel with both steps of the global alignment step and the second stage selection step.

(2) The method for designing a primer used for a polymerase chain reaction according to the (1), the method further comprising: a first target region selection step of selecting a first target region to be amplified through the polymerase chain reaction, from regions on a genome; a first primer candidate base sequence generation step of generating at least one base sequence of a primer candidate for amplifying the first target region based on each base sequence in vicinity regions at both ends of the first target region on the genome; a first local alignment step of obtaining a local alignment score by performing pairwise local alignment on the base sequence of the primer candidate under a condition that a partial sequence to be subjected to comparison includes the 3' terminal of the base sequence of the primer candidate; a first step of first stage selection of performing first stage selection of the base sequence of the primer candidate based on the local alignment score obtained in the local alignment step; a first global alignment step of obtaining a global alignment score by performing pairwise global alignment on a base sequence which is of an up to three base length and includes the 3' terminal of the base sequence of the primer candidate; a first step of second stage selection of performing second stage selection of the base sequence of the primer candidate based on the global alignment score obtained in the global alignment step; a first primer employment step of employing the base sequence of the primer candidate which has been selected in both of the first step of first stage selection and the first step of second stage selection as a base sequence of a primer for amplifying the first target region; a second target region selection step of selecting a second target region to be amplified through the polymerase chain reaction, from regions on a genome; a second primer candidate base sequence generation step of generating at least one base sequence of a primer candidate for amplifying the second target region based on each of the base sequences in vicinity regions at both ends of the second target region on the genome; a second local alignment step of obtaining a local alignment score by performing pairwise local alignment on the base sequence of the primer candidate for amplifying the second target region and the base sequence of the primer which has already been employed, under a condition that partial sequences to be subjected to comparison include the 3' terminal of the base sequence of the primer candidate and the 3' terminal of the base sequence of the primer which has already been employed; a second step of first stage selection of performing first stage selection of the base sequence of the primer candidate for amplifying the second target region based on the local alignment score; a second global alignment step of obtaining a global alignment score by performing pairwise global alignment on base sequences which are of an up to three base length and include the 3' terminal of the base sequence of the primer candidate for amplifying the second target region and the 3' terminal of the base sequence of the primer which has already been employed; a second step of second stage selection of performing second stage selection of the base sequence of the primer candidate for amplifying the second target region based on the global alignment score; and a second primer employment step of employing the base sequence of the primer candidate which has been selected in both of the second step of first stage selection and the second step of second stage selection as a base sequence of a primer for amplifying the second target region, in which both steps of the first local alignment step and the first step of first stage selection are performed before or after both steps of the first global alignment step and the first step of second stage selection, or performed in parallel with both steps of the first global alignment step and the first step of second stage selection, both steps of the second local alignment step and the second step of first stage selection are performed before or after both steps of the second global alignment step and the second step of second stage selection, or performed in parallel with both steps of the second global alignment step and the second step of second stage selection, and, in a case where there are three or more of the target regions, the steps from the second target region selection step to the second primer employment step are repeated with respect to all of the target regions until a base sequence of a primer for amplifying each target region is employed.

(3) Described herein is also a primer set used for a polymerase chain reaction, in which a local alignment score obtained by performing pairwise local alignment on a base sequence of each primer under a condition that a partial sequence to be subjected to comparison includes the 3' terminal of the base sequence is less than a first threshold value, and a global alignment score obtained by performing pairwise global alignment on a base sequence which has a predetermined number of bases and the 3' terminal of the base sequence of each primer is less than a second threshold value.

According to the present invention, it is possible to provide a method for designing a primer used for a polymerase chain reaction which can selectively amplify an objective gene region efficiently.

In addition, the primer set described herein is a primer set used for a polymerase chain reaction which can selectively amplify an objective gene region efficiently.

## BRIEF DESCRIPTION OF THE DRAWINGS

Fig. 1 is a block diagram of a method for designing a primer of the present invention.

Fig. 2 is a view indicating local alignment of a pair of a base sequence represented by SEQ ID No: 1 and a base sequence represented by SEQ ID No: 2 in Example 1.

Fig. 3 is a view indicating local alignment of a pair of the base sequence represented by SEQ ID No: 1 and a base sequence represented by SEQ ID No: 3 in Example 1.

Fig. 4 is a view indicating local alignment of a pair of the base sequence represented by SEQ ID No: 1 and a base sequence represented by SEQ ID No: 4 in Example 1.

Fig. 5 is a view indicating local alignment of a pair of the base sequence represented by SEQ ID No: 1 and a base sequence represented by SEQ ID No: 5 in Example 1.

Fig. 6 is a view indicating local alignment of a pair of the base sequence represented by SEQ ID No: 1 and a base sequence represented by SEQ ID No: 6 in Example 1.

Fig. 7 is a view indicating local alignment of a pair of the base sequence represented by SEQ ID No: 1 and a base sequence represented by SEQ ID No: 7 in Example 1.

Fig. 8 is a view indicating local alignment of a pair of the base sequence represented by SEQ ID No: 1 and a base sequence represented by SEQ ID No: 8 in Example 1.

Fig. 9 is a view indicating local alignment of a pair of the base sequence represented by SEQ ID No: 2 and the base sequence represented by SEQ ID No: 3 in Example 1.

Fig. 10 is a view indicating local alignment of a pair of the base sequence represented by SEQ ID No: 2 and the base sequence represented by SEQ ID No: 4 in Example 1.

Fig. 11 is a view indicating local alignment of a pair of the base sequence represented by SEQ ID No: 2 and the base sequence represented by SEQ ID No: 5 in Example 1.

Fig. 12 is a view indicating local alignment of a pair of the base sequence represented by SEQ ID No: 2 and the base sequence represented by SEQ ID No: 6 in Example 1.

Fig. 13 is a view indicating local alignment of a pair of the base sequence represented by SEQ ID No: 2 and the base sequence represented by SEQ ID No: 7 in Example 1.

Fig. 14 is a view indicating local alignment of a pair of the base sequence represented by SEQ ID No: 2 and the base sequence represented by SEQ ID No: 8 in Example 1.

Fig. 15 is a view indicating local alignment of a pair of the base sequence represented by SEQ ID No: 3 and the base sequence represented by SEQ ID No: 4 in Example 1.

Fig. 16 is a view indicating local alignment of a pair of the base sequence represented by SEQ ID No: 3 and the base sequence represented by SEQ ID No: 5 in Example 1.

Fig. 17 is a view indicating local alignment of a pair of the base sequence represented by SEQ ID No: 3 and the base sequence represented by SEQ ID No: 6 in Example 1.

Fig. 18 is a view indicating local alignment of a pair of the base sequence represented by SEQ ID No: 3 and the base sequence represented by SEQ ID No: 7 in Example 1.

Fig. 19 is a view indicating local alignment of a pair of the base sequence represented by SEQ ID No: 3 and the base sequence represented by SEQ ID No: 8 in Example 1.

Fig. 20 is a view indicating local alignment of a pair of the base sequence represented by SEQ ID No: 4 and the base sequence represented by SEQ ID No: 5 in Example 1.

Fig. 21 is a view indicating local alignment of a pair of the base sequence represented by SEQ ID No: 4 and the base sequence represented by SEQ ID No: 6 in Example 1.

Fig. 22 is a view indicating local alignment of a pair of the base sequence represented by SEQ ID No: 4 and the base sequence represented by SEQ ID No: 7 in Example 1.

Fig. 23 is a view indicating local alignment of a pair of the base sequence represented by SEQ ID No: 4 and the base sequence represented by SEQ ID No: 8 in Example 1.

Fig. 24 is a view indicating local alignment of a pair of the base sequence represented by SEQ ID No: 5 and the base sequence represented by SEQ ID No: 6 in Example 1.

Fig. 25 is a view indicating local alignment of a pair of the base sequence represented by SEQ ID No: 5 and the base sequence represented by SEQ ID No: 7 in Example 1.

Fig. 26 is a view indicating local alignment of a pair of the base sequence represented by SEQ ID No: 5 and the base sequence represented by SEQ ID No: 8 in Example 1.

Fig. 27 is a view indicating local alignment of a pair of the base sequence represented by SEQ ID No: 6 and the base sequence represented by SEQ ID No: 7 in Example 1.

Fig. 28 is a view indicating local alignment of a pair of the base sequence represented by SEQ ID No: 6 and the base sequence represented by SEQ ID No: 8 in Example 1.

Fig. 29 is a view indicating local alignment of a pair of the base sequence represented by SEQ ID No: 7 and the base sequence represented by SEQ ID No: 8 in Example 1.

Fig. 30 is a view indicating global alignment of two bases at the 3' terminal of a pair of arbitrary two base sequences selected from the base sequences represented by SEQ ID No: 1 to 8 in Example 1.

Fig. 31 is a view indicating local alignment of a pair of a base sequence represented by SEQ ID No: 9 and a base sequence represented by SEQ ID No: 10 in Comparative Example 1.

Fig. 32 is a view indicating local alignment of a pair of a base sequence represented by SEQ ID No: 11 and a base sequence represented by SEQ ID No: 12 in Comparative Example 2.

Fig. 33 is a view indicating global alignment of two bases at the 3' terminal of a pair of the base sequence represented by SEQ ID No: 11 and the base sequence represented by SEQ ID No: 12 in Comparative Example 2.

## DESCRIPTION OF THE PREFERRED EMBODIMENTS

First, advantageous points of the present invention compared with the related art will be described.

The technology disclosed in WO2004/081225A attempts to provide amplification means, which is not deviated with respect to the whole genome region by providing a universal primer. An example of an advantageous point in the present invention with respect to the related art disclosed in WO2004/081225A includes a point that it is possible to selectively amplify an objective gene region efficiently in the present invention whereas the technology disclosed in WO2004/081225A does not selectively amplify a specific gene region. In addition, the technology disclosed in WO2008/004691A attempts to design a primer set in which a primer dimer is hardly formed by performing local alignment on the entire primer base sequence and selecting primers in which complementarity of the entire sequence is low. An example of an advantageous point of the present invention with respect to the related art disclosed in WO2008/004691A includes a point that it is possible to selectively amplify an objective gene region efficiently since the complementarity of the entire sequence including the 3' terminal is decreased through local alignment and a primer group is generated such that the complementarity of an extremely short partial sequence at the 3' terminal having a length, for example, about 5 nucleotides or shorter is decreased through global alignment in the present invention whereas it is impossible to sufficiently prevent the formation of the primer dimer only by decreasing the complementarity of the entire sequence.

Hereinafter, the present invention will be described in detail.

[Method for Designing Primer Used for Polymerase Chain Reaction (First Embodiment)]

A first embodiment of the method for designing a primer used for a polymerase chain reaction of the present invention includes: (a) a target region selection step of selecting a target region to be amplified through the above-described polymerase chain reaction, from regions on a genome; (b) a primer candidate base sequence generation step of generating at least one base sequence of a primer candidate for amplifying the above-described target region based on each base sequence in vicinity regions at both ends of the above-described target region on the genome; (c) a local alignment step of obtaining a local alignment score by performing pairwise local alignment on the base sequence of the above-described primer candidate under a condition that a partial sequence to be subjected to comparison includes the 3' terminal of the base sequence of the above-described primer candidate; (d) a first stage selection step of performing first stage selection of the base sequence of the above-described primer candidate based on the local alignment score obtained in the above-described (c) local alignment step; (e) a global alignment step of obtaining a global alignment score by performing pairwise global alignment on a base sequence which is of an up to three base length and includes the 3' terminal of the base sequence of the above-described primer candidate; (f) a second stage selection step of performing second stage selection of a base sequence of the above-described primer candidate based on the global alignment score obtained in the above-described (e) global alignment step; and (g) a primer employment step of employing the base sequence of the primer candidate which has been selected in both of the above-described (d) first stage selection step and the above-described (f) second stage selection step as a base sequence of a primer for amplifying the above-described target region, in which both steps of the above-described local alignment step and the above-described first stage selection step are performed before or after both steps of the above-described global alignment step and the above-described second stage selection step, or performed in parallel with both steps of the above-described global alignment step and the above-described second stage selection step.

Each step of the first embodiment of the method for designing a primer used for a polymerase chain reaction of the present invention will be described in detail.

(a) Target Region Selection Step

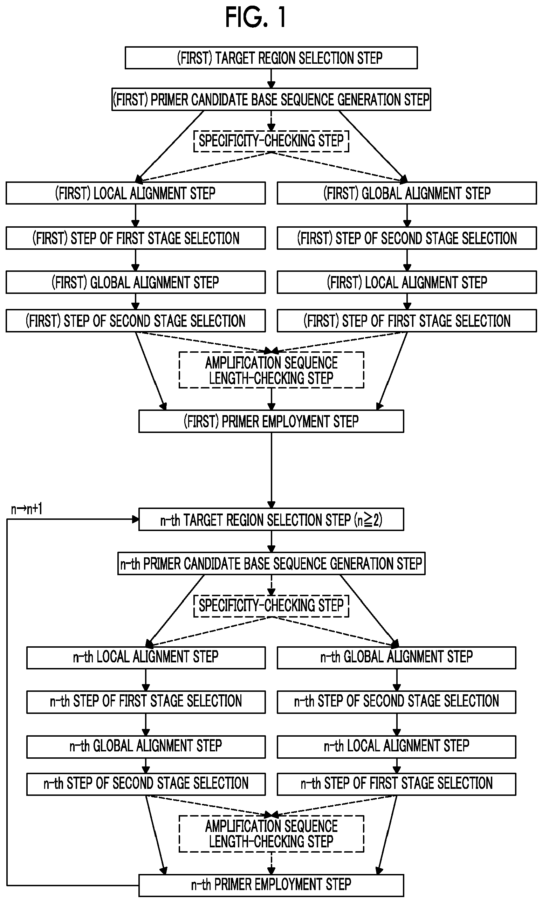

The target region selection step is shown in a block diagram of Fig. 1 as "(FIRST) TARGET REGION SELECTION STEP".

The target region selection step is a step of selecting a target region to be amplified through a polymerase chain reaction, from regions on a genome.

(Regions on Genome)

The "regions on a genome" in the present invention refers to a region on genomic DNA in which a site relating to genetic polymorphism, a single gene disease, a multifactorial disease, cancer, or the like exists. Here, the length of a region is not particularly limited, and may be one or more bases.

The regions on a genome from which a target region is selected may exist in either a gene region or a non-gene region. Here, the gene region includes: a coding region in which gene encoding proteins, a ribosomal ribonucleic acid (RNA) gene, a transfer RNA gene, and the like exist; and a non-coding region in which an intron dividing a gene, a transcription regulatory region, a 5' leader sequence, a 3' leader sequence, and the like exist. In addition, the non-gene region includes: a non-repetitive sequence such as a pseudogene, a spacer, a response element, and a replication origin; and a repetitive sequence such as a tandem repetitive sequence and an interspersed repetitive sequence.

Examples of genetic polymorphism include single nucleotide polymorphism (SNP), single nucleotide variant (SNV), short tandem repeat polymorphism (STRP), mutation, and insertion and/or deletion (indel).

The single gene disease is a disease caused by single gene abnormality. Examples of the abnormality include deletion or duplication of the gene, and/or substitution of a base in a gene, and insertion and/or deletion. A single gene that causes a single gene disease is called a "responsible gene".

The multifactorial disease is a disease in which a plurality of genes are involved in the onset. In some cases, a specific combination or the like of SNP may be related thereto. These genes are called "sensitive genes" in the sense that the genes are susceptible to a disease.

Cancer is a disease caused by gene mutation. Similarly to other diseases, there is hereditary (familial) cancer which is called a hereditary tumor (familial tumor) or the like.

The number of regions on a genome is not particularly limited. This is because regions on a genome are a candidate list in a case of selecting a target region, and it is unnecessary to design a primer for all the regions even if a large number of regions is listed.

(Target Region)

The target region is a region selected as an object to be amplified through a polymerase chain reaction from the above-described regions on a genome. Here, the purpose of selection is not limited to detection of genetic polymorphism, diseases, cancer, or the like related to each region, and may be detection of aneuploidy of a chromosome or the like. In addition, the number of purposes of the selection is not limited to one, and may be two or more.

The number of regions on a genome to be selected as target regions varies depending on the purpose. The number of regions thereof is not particularly limited as long as it is greater than or equal to one region. In general, the number of regions thereof is preferably greater than or equal to 3 regions, more preferably greater than or equal to 5 regions, and still more preferably greater than or equal to 10 regions.

(Polymerase Chain Reaction)

In the present invention, the polymerase chain reaction (PCR) is a reaction for synthesizing DNA from template DNA using DNA polymerase. Unlike intracellular DNA synthesis, one or more oligonucleotides, in general, two or more oligonucleotides which are called primers are required for synthesizing DNA in PCR. In some cases, a combination of primers simultaneously used in a PCR reaction system is referred to as a primer set.

PCR can be easily extended from a simple system in which a region is amplified using a primer set which is a pair to a complex system (multiplex PCR) in which a plurality of regions are simultaneously amplified using a plurality of pairs of primer sets.

The advantage of PCR is that it is possible to selectively amplify only a desired region from extremely long DNA molecules of a human genome (3 billion base pairs). In addition, it is possible to obtain a sufficient amount of an amplification product of a desired region using an extremely trace amount of genomic DNA as a template.

In addition, another example of an advantage of PCR includes a short period of time of about 2 hours generally required for the amplification even though the period of time depends on the protocols.

Still another example of the advantage of PCR is that the process is simple, and therefore, it is possible to perform the amplification using a fully automated desktop device.

(b) Primer Candidate Base Sequence Generation Step

The primer candidate base sequence generation step is shown in the block diagram of Fig. 1 as "(FIRST) PRIMER CANDIDATE BASE SEQUENCE GENERATION STEP".

The primer candidate base sequence generation step is a step of generating at least one base sequence of a primer candidate for amplifying a target region based on each base sequence in vicinity regions at both ends of the target region on a genome.

The vicinity regions of the target region are collectively called regions on the outside of the 5' terminal of the target region and regions on the outside of the 3' terminal of the target region. The inside of the target region is not included in the vicinity regions.

The length of a vicinity region is not particularly limited, but is preferably less than or equal to a length that can be expanded through PCR and more preferably less than or equal to the upper limit of a fragment length of DNA for which amplification is desired. A length facilitating application of concentration selection and/or sequence reading is particularly preferable. The length of a vicinity region may be appropriately changed in accordance with the type of enzyme (DNA polymerase) used for PCR. The specific length of a vicinity region is preferably about 20 to 500 bases, more preferably about 20 to 300 bases, still more preferably about 20 to 200 bases, and particularly preferably about 50 to 200 bases.

In addition, in a case of generating a base sequence of a primer candidate, points, such as the length of a primer, the GC content (referring to a total mole percentage of guanine (G) and cytosine (C) in all nucleic acid bases), a Tm value (which is a temperature at which 50% of double-stranded DNA is dissociated and becomes single-stranded DNA, and in which Tm is derived from a melting temperature), and deviation of a sequence, to be taken into consideration in a general method for designing a primer are the same.

The length of a primer (the number of nucleotides) is not particularly limited, but is preferably 15 mer to 45 mer, more preferably 15 mer to 35 mer, still more preferably 15 mer to 25 mer, and particularly preferably 15 mer to 20 mer. In a case where the length of a primer is within this range, it is easy to design a primer excellent in specificity and amplification efficiency.

The GC content is not particularly limited, but is preferably 40 mol% to 60 mol% and more preferably 45 mol% to 55 mol%. In a case where the GC content is within this range, a problem such as a decrease in the specificity and the amplification efficiency due to a high-order structure is less likely to occur.

The Tm value is not particularly limited, but is preferably within a range of 50°C to 65°C and more preferably within a range of 55°C to 65°C.

The Tm value can be calculated using software such as OLIGO Primer Analysis Software (manufactured by Molecular Biology Insights) or Primer3 (http://www-genome.wi.mit.edu/ftp/distribution/software/).

In addition, the Tm value can also be obtained through calculation using the following formula from the number of A's, T's, G's, and C's (which are respectively set as nA, nT, nG, and nC) in a base sequence of a primer.

$$\text{Tm value (°C)} = 2(nA + nT) + 4(nC + nG)$$

The method for calculating the Tm value is not limited thereto and can be calculated through various well-known methods in the related art.

The base sequence of a primer candidate is preferably set as a sequence in which there is no deviation of bases as a whole. For example, it is desirable to avoid a GC-rich sequence and a partial AT-rich sequence.

In addition, it is also desirable to avoid continuation of T and/or C (polypyrimidine) and continuation of A and/or G (polypurine).

Furthermore, it is preferable that a 3' terminal base sequence avoids a GC-rich sequence or an AT-rich sequence. G or C is preferable for a 3' terminal base, but is not limited thereto.

(Specificity-Checking Step)

If desired, a specificity-checking step of evaluating specificity of a base sequence of a primer candidate may be performed based on sequence complementarity with respect to genomic DNA of a base sequence of each primer candidate which has been generated in the above-described (b) Primer Candidate Base Sequence Generation Step.

In the specificity check, in a case where local alignment of a base sequence of genomic DNA and a base sequence of a primer candidate is performed and a local alignment score is less than a predetermined value, it is possible to evaluate that the complementarity of the base sequence of the primer candidate with respect to genomic DNA is low and the specificity of the base sequence of the primer candidate with respect to genomic DNA is high. Here, it is desirable to perform local alignment on also a complementary chain of genomic DNA. This is because genomic DNA is double-stranded whereas the primer is single-stranded DNA. In addition, a base sequence complementary to the base sequence of the primer candidate may be used instead of the base sequence of the primer candidate. The complementarity can be considered as homology with respect to a complementary chain.

In addition, homology search may be performed on genomic DNA base sequence database using the base sequence of the primer candidate as a query sequence. Examples of a homology search tool include Basic Local Alignment Search Tool (BLAST) (Altschul, S. A., et al., "Basic Local Alignment Search Tool", Journal of Molecular Biology, 1990, October, Vol. 215, pp. 403-410) and FASTA (Pearson, W. R., et al., "Improved tools for biological sequence comparison", Proceedings of the National Academy of Sciences of the United States of America, National Academy of Sciences, 1988, April, Vol. 85, pp. 2444-2448). It is possible to obtain local alignment as a result of performing the homology search.

All of the scoring system and a threshold value of a local alignment score are not particularly limited, and can be appropriately set in accordance with the length of a base sequence of a primer candidate and/or PCR conditions, and the like. In a case of using a homology search tool, a default value of the homology search tool may be used.

For example, as the scoring system, it is considered that complementary base (match) = +1, non-complementary base (mismatch) = -1, and insertion and/or deletion (gap penalty) = -3 are employed and the threshold value is set to be +15.

In a case where a base sequence of a primer candidate has complementarity to a base sequence at an unexpected position on genomic DNA but has low specificity thereto, in some cases, an artifact is amplified instead of a target region in a case where PCR is performed using a primer of the base sequence of a primer candidate. Therefore, the case where the base sequence of the primer candidate has complementarity to the base sequence at an unexpected position on genomic DNA but has low specificity thereto is excluded.

(c) Local Alignment Step

The local alignment step is shown in the block diagram of Fig. 1 as "(FIRST) LOCAL ALIGNMENT STEP".

The local alignment step is a step of obtaining a local alignment score by performing pairwise local alignment on all pairs each consisting of two base sequences extracted from the base sequences of the primer candidates which have been generated in the above-described (b) Primer Candidate Base Sequence Generation Step and is used for amplifying a target region, under a condition that partial sequences to be subjected to comparison include the 3' terminals of the base sequences of the primer candidates.

A combination of pairs of base sequences to be subjected to local alignment may be a combination selected while allowing overlapping, or may be a combination selected without allowing overlapping. However, in a case where formability of a primer dimer between primers of an identical base sequence has not yet been evaluated, the combination selected while allowing overlapping is preferable.

The total number of combinations is "$_mH_2 = {_{m+1}C_2} = (m+1)! / 2(m-1)!$" in a case where the selection is performed while allowing overlapping, and is "$_mC_2 = m(m-1) / 2$" in a case where the selection is performed without allowing overlapping, in which the number of base sequences which have been generated in the above-described (b) Primer Candidate Base Sequence Generation Step is set to be m.

In a case where both steps of (e) Global Alignment Step and (f) Second Stage Selection Step to be described below are performed first, the present step and (d) First Stage Selection Step to be described below may be performed on primer candidates selected in (f) Second Stage Selection Step.

Local alignment is alignment which is performed on a partial sequence and in which it is possible to locally check a portion with high complementarity.

However, in the present invention, the local alignment is different from local alignment usually performed on a base sequence, and is designed such that partial sequences to be subjected to comparison include the 3' terminals of both base sequences by performing local alignment under the condition that the "partial sequences to be subjected to comparison include the 3' terminals of the base sequences". Furthermore, in the present invention, an embodiment is preferable in which partial sequences to be subjected to comparison include the 3' terminals of both base sequences by performing local alignment under the condition that the "partial sequences to be subjected to comparison include the 3' terminals of the base sequences", that is, the condition that "only alignments in which a partial sequence to be subjected to comparison begins at the 3' terminal of one sequence and ends at the 3' terminal of the other sequence".

Local alignment may be performed by inserting a gap. The gap means insertion and/or deletion (indel) of a base.

In addition, in the local alignment, a case where bases are complementary to each other between base sequence pairs is regarded as a match and a case where bases are not complementary to each other therebetween is regarded as a mismatch.

Alignment is performed such that scores for each of the match, the mismatch, and the indel are given and the total score becomes a maximum. The score may be appropriately set. For example, a scoring system may be set as in the following Table 1. "-" in Table 1 represents a gap (insertion and/or deletion (indel)).

[Table 1]

Table 1

|   | A | T | G | C | - |
|---|---|---|---|---|---|
| A | -1 | +1 | -1 | -1 | -3 |
| T | +1 | -1 | -1 | -1 | -3 |
| G | -1 | -1 | -1 | +1 | -3 |
| C | -1 | -1 | +1 | -1 | -3 |
| - | -3 | -3 | -3 | -3 |  |

"-": gap (indel)

For example, it is considered that local alignment is pertormed on base sequences of SEQ ID No: 1 and SEQ ID No: 2 shown in the following Table 2. Here, the scoring system is as shown in Table 1.

[Table 2]

Table 2

| | Base sequence (5' → 3') |
|---|---|
| SEQ ID No: 1: | GCTTGGCCTTGGGAATGTGG |
| SEQ ID No: 2: | GGCAATATGGCCAATGATGG |

From the base sequences of SEQ ID No: 1 and SEQ ID No: 2, a dot matrix shown in Table 3 is generated. Specifically, the base sequence of SEQ ID No: 1 is arranged from the left to the right in an orientation of 5' to 3' and the base sequence of SEQ ID No: 2 is arranged from the bottom to the top in an orientation of 5' to 3'. "•" is filled in a grid of which bases are complementary to each other, and a dot matrix shown in Table 3 is obtained.

[Table 3]

Table 3

SEQ ID No: 1 →

| | G | C | T | T | G | G | C | C | T | T | G | G | G | A | A | T | G | T | G | G |
|---|---|---|---|---|---|---|---|---|---|---|---|---|---|---|---|---|---|---|---|---|
| G | | • | | | | | • | • | | | | | | | | | | | | |
| G | | • | | | | | • | • | | | | | | | | | | | | |
| T | | | | | | | | | | | | | | • | • | | | | | |
| A | | | • | • | | | | | • | • | | | | | | | | • | | |
| G | | • | | | | | • | • | | | | | | | | | | | | |
| T | | | | | | | | | | | | | | • | • | | | | | |
| A | | | • | • | | | | | • | • | | | | | | | | • | | |
| A | | | • | • | | | | | • | • | | | | | | | | • | | |
| G | | • | | | | | • | • | | | | | | | | | | | | |
| G | | • | | | | | • | • | | | | | | | | | | | | |
| T | | | | | | | | | | | | | | • | • | | | | | |
| A | | | • | • | | | | | • | • | | | | | | | | • | | |
| T | | | | | | | | | | | | | | • | • | | | | | |
| A | | | • | • | | | | | • | • | | | | | | | | • | | |
| A | | | • | • | | | | | • | • | | | | | | | | • | | |
| C | • | | | | • | • | | | | | • | • | • | | | | • | | • | • |
| G | | • | | | | | • | • | | | | | | | | | | | | |
| G | | • | | | | | • | • | | | | | | | | | | | | |

(SEQ ID No: 2 →)

From the dot matrix shown in Table 3, Alignment (pairwise alignment) of partial sequences shown in the following Table 4 is obtained (refer to a diagonal line portion of Table 3).

[Table 4]

Table 4

| Partial sequence from SEQ ID No: 1: | 5'- | G | A | A | T | G | T | G | G | -3' |
| Partial sequence from SEQ ID No: 2: | | | | | | | | | | | |
| | | | | │ | │ | | | | | |
| | 3'- | G | G | T | A | G | T | A | A | -5' |

Due to match (+1) x 2, mismatch (-1) x 6, and indel (-3) x 0, the local alignment score is "-4".

The alignment (pairwise alignment) can be obtained not only through the dot matrix method exemplified herein, but also through a dynamic programming method, a word method, or various other methods.

(d) First Stage Selection Step

The first stage selection step is shown in the block diagram of Fig. 1 as "(FIRST) STEP OF FIRST STAGE SELECTION".

The first stage selection step is a step of performing first stage selection of base sequences of primer candidates which have been generated in the (b) Primer Candidate Base Sequence Generation Step, based on the local alignment score obtained in the above-described (c) Local Alignment Step.

A threshold value (first threshold value) of the local alignment score is predetermined.

In a case where a local alignment score of a pair of two base sequences is less than the first threshold value, it is determined that the pair of these two base sequences has low dimer formability, and the following step is performed. In contrast, in a case where a local alignment score of a pair of two base sequences is greater than or equal to the first threshold value, it is determined that the pair of these two base sequences has high dimer formability, and the following step is not performed on the pair.

The first threshold value is not particularly limited and can be appropriately set. For example, the first threshold value may be set using a PCR condition such as the amount of genomic DNA which becomes a template for a polymerase chain reaction.

Here, in the example in which the above-described (c) Local Alignment Step is shown, a case where the first threshold value is set to "3" is considered.

In the above-described example, the local alignment score is "-3" and is less than "3" which is the first threshold value. Therefore, it is possible to determine that the pair of the base sequences of SEQ ID No: 1 and SEQ ID No: 2 has low dimer formability.

The present step is performed on all of the pairs for which scores are calculated in the above-described (c) Local Alignment Step.

(e) Global Alignment Step

The global alignment step is shown in the block diagram of Fig. 1 as "(FIRST) GLOBAL ALIGNMENT STEP".

The global alignment step is a step of obtaining a global alignment score by performing pairwise global alignment on a base sequence which has a predetermined sequence length and includes the 3' terminal of the base sequence of the primer candidate regarding all pairs each consisting of two base sequences extracted from the base sequences of the primer candidates which have been generated in the above-described (b) Primer Candidate Base Sequence Generation Step and is used for amplifying a target region. In this invention, the predetermined sequence length is an up to three base length.

A combination of pairs of base sequences to be subjected to global alignment may be a combination selected while allowing overlapping, or may be a combination selected without allowing overlapping. However, in a case where formability of a primer dimer between primers of an identical base sequence has not yet been evaluated, the combination selected while allowing overlapping is preferable.

The total number of combinations is "$_mH_2 = {}_{m+1}C_2 = (m+1)! / 2(m-1)!$" in a case where the selection is performed while allowing overlapping, and is "$_mC_2 = m(m-1) / 2$" in a case where the selection is performed without allowing overlapping, in which the number of base sequences which have been generated in the above-described (b) Primer Candidate Base Sequence Generation Step is set to be m.

In a case where both steps of (c) Local Alignment Step and (d) First Stage Selection Step which have been described above are performed first, the present step and (f) Second Stage Selection Step to be described below may be performed on primer candidates selected in (d) First Stage Selection Step.

Global alignment is an alignment which is performed on the entire sequence and in which it is possible to check complementarity of the entire sequence.

However, here, the "entire sequence" refers to the entirety of a base sequence which is of an up to three base length and includes the 3' terminal of a base sequence of a primer candidate.

Global alignment may be performed by inserting a gap. The gap means insertion and/or deletion (indel) of a base.

In addition, in the global alignment, a case where bases are complementary to each other between base sequence pairs is regarded as a match and a case where bases are not complementary to each other therebetween is regarded as a mismatch.

Alignment is performed such that scores for each of the match, the mismatch, and the indel are given and the total score becomes a maximum. The score may be appropriately set. For example, a scoring system may be set as in Table 1 described above. "-" in Table 1 represents a gap (insertion and/or deletion (indel)).

For example, it is considered that global alignment is performed on three bases (refer to portions with capital letters and correspond to the "base sequence which has a predetermined sequence length and includes the 3' terminal") at the 3' terminal of each base sequence of SEQ ID No: 1 and SEQ ID No: 2 shown in the following Table 5. Here, the scoring system is as shown in Table 1.

[Table 5]

| | Table 5 |
| --- | --- |
| | Base sequence (5' → 3') |
| SEQ ID No: 1: | gcttggccttgggaatgTGG |
| SEQ ID No: 2: | ggcaatatggccaatgaTGG |

In a case of performing global alignment on base sequences of the three bases (portion with capital letters) at the 3' terminal of the base sequence of SEQ ID No: 1 and the three bases (portion with capital letters) at the 3' terminal of SEQ ID No: 2 such that the score becomes a maximum, it is possible to obtain alignment (pairwise alignment) shown in the following Table 6.

[Table 6]

| | Table 6 | | | |
| --- | --- | --- | --- | --- |
| Three bases at 3' terminal of SEQ ID No: 1: | 5'- | T | G | G | -3' |
| Three bases at 3' terminal of SEQ ID No: 2: | 3'- | G | G | T | -5' |

As match (+1) x 0, mismatch (-1) x 3, and indel (-3) x 0, the global alignment score is "-3".

The alignment (pairwise alignment) can be obtained through the dot matrix method, a dynamic programming method, a word method, or various other methods.

(f) Second Stage Selection Step

The second stage selection step is shown in the block diagram of Fig. 1 as "(FIRST) STEP OF SECOND STAGE SELECTION".

The second stage selection step is a step of performing second stage selection of base sequences of primer candidates which have been generated in the above-described (b) Primer Candidate Base Sequence Generation Step based on the global alignment score obtained in the above-described (e) global alignment step.

A threshold value (second threshold value) of the global alignment score is predetermined.

In a case where a global alignment score of a pair of two base sequences is less than the second threshold value, it is determined that the pair of these two base sequences has low dimer formability, and the following step is performed. In contrast, in a case where a global alignment score of a pair of two base sequences is greater than or equal to the second threshold value, it is determined that the pair of these two base sequences has high dimer formability, and the following step is not performed on the pair.

The second threshold value is not particularly limited and can be appropriately set. For example, the second threshold value may be set using a PCR condition such as the amount of genomic DNA which becomes a template for a polymerase chain reaction.

It is possible to set the global alignment score obtained by performing pairwise global alignment on a base sequence which has a predetermined number of bases, i.e. a length of up to three bases, and includes the 3' terminal of a base sequence of each primer to be less than the second threshold value by setting a base sequence with several bases from the 3' terminal of a primer as an identical base sequence.

Here, in the example in which the above-described (e) Global Alignment Step is shown, a case where the second threshold value is set to "3" is considered.

In the above-described example, the global alignment score is "-3" and is less than "3" which is the second threshold value. Therefore, it is possible to determine that the pair of the base sequences of SEQ ID No: 1 and SEQ ID No: 2 has low dimer formability.

The present step is performed on all of the pairs for which scores are calculated in the above-described (e) Global Alignment Step.

Both steps of the above-described (c) Local Alignment Step and the above-described (d) First Stage Selection Step may be performed before or after both steps of the above-described (e) Global Alignment Step and the above-described (f) Second Stage Selection Step, or may be performed in parallel with both steps of the above-described (e) Global Alignment Step and the above-described (f) Second Stage Selection Step.

In addition, in order to reduce the amount of calculation, it is preferable to perform both steps of the above-described (c) Local Alignment Step and the above-described (d) First Stage Selection Step in a combination which has passed the above-described (f) Second Stage Selection Step after first performing both steps of the above-described (e) Global Alignment Step and the above-described (f) Second Stage Selection Step. Particularly, as the number of target regions and the number of base sequences of primer candidates are increased, the effect of reducing the amount of calculation is increased, and it is possible to speed up the overall processing.

This is because the amount of calculation of a global alignment score is smaller than that of a local alignment score which is obtained by searching a partial sequence with high complementarity from the entire base sequence under the condition that the base sequence includes the 3' terminal and it is possible to speed up the processing since global alignment is performed on a base sequence with a short length, namely an up to three base length, called a "predetermined sequence length" in the above-described (e) Global Alignment Step. It is known that the global alignment is faster than the local alignment in a case of alignment with respect to a sequence having an identical length in a well-known algorithm.

(Amplification Sequence Length-Checking Step)

If desired, an amplification sequence length-checking step of calculating the distance between ends of base sequences of primer candidates for which it has been determined that formability of a primer dimer is low in the above-described (d) First Stage Selection Step and the above-described (f) Second Stage Selection Step, on genomic DNA or chromosomal DNA regarding pairs of the base sequences of the primer candidates, and determining whether the distance is within a predetermined range may be performed.

In a case where the distance between the ends of the base sequences is within the predetermined range, it is possible to determine that there is a high possibility that the pairs of the base sequences of the primer candidates can appropriately amplify a target region. The distance between the ends of the base sequences of the primer candidates is not particularly limited, and can be appropriately set in accordance with the PCR condition such as the type of enzyme (DNA polymerase). For example, the distance between the ends of the base sequences of the primer candidates can be set to be within various ranges such as a range of 100 to 200 bases (pair), a range of 120 to 180 bases (pair), a range of 140 to 180 bases (pair) a range of 140 to 160 bases (pair), and a range of 160 to 180 bases (pair).

(g) Primer Employment Step

The primer employment step is shown in the block diagram of Fig. 1 as "(FIRST) PRIMER EMPLOYMENT STEP".

The primer employment step is a step of employing a base sequence of a primer candidate which has been selected in both of the above-described (d) First Stage Selection Step and the above-described (f) Second Stage Selection Step, as a base sequence of a primer for amplifying the above-described target region.

That is, in the present step, a base sequence of a primer candidate, in which a local alignment score obtained by performing pairwise local alignment on a base sequence of each primer candidate under a condition that a partial sequence to be subjected to comparison includes the 3' terminal of the base sequence is less than the first threshold value, and a global alignment score obtained by performing pairwise global alignment on a base sequence which has a predetermined number of bases, i.e. a length of up to three bases, and includes the 3' terminal of the base sequence of each primer candidate is less than the second threshold value, is employed as a base sequence of a primer for amplifying a target region.

For example, it is considered that base sequences of SEQ ID No: 1 and SEQ ID No: 2 shown in Table 7 are employed as base sequences of primers for amplifying a target region.

[Table 7]

Table 7

| | Base sequence (5' → 3') |
|---|---|
| SEQ ID No: 1: | GCTTGGCCTTGGGAATGTGG |
| SEQ ID No: 2: | GGCAATATGGCCAATGATGG |

As already described, the local alignment score is "-3" and is less than "3" which is the first threshold value. Moreover, the global alignment score is "-3" and is less than "3" which is the second threshold value.

Accordingly, it is possible to employ the base sequence of the primer candidate represented by SEQ ID No: 1 and the base sequence of primer candidate represented by SEQ ID No: 2 as base sequences of primers for amplifying a target region.

[Method for Designing Primer Used for Polymerase Chain Reaction (Second Embodiment)]

A second embodiment of a method for designing a primer used for a polymerase chain reaction of the invention includes the following steps: ($a_1$) A first target region selection step of selecting a first target region to be amplified through the polymerase chain reaction, from regions on a genome; ($b_1$) a first primer candidate base sequence generation step of generating at least one base sequence of a primer candidate for amplifying the above-described first target region based on each base sequence in vicinity regions at both ends of the above-described first target region on the genome; ($c_1$) a first local alignment step of obtaining a local alignment score by performing pairwise local alignment on the base sequence of the above-described primer candidate under a condition that a partial sequence to be subjected to comparison includes the 3' terminal of the base sequence of the above-described primer candidate; ($d_1$) a first step of first stage selection of performing first stage selection of the base sequence of the above-described primer candidate based on the local alignment score obtained in the above-described ($c_1$) first local alignment step; ($e_1$) a first global alignment step of obtaining a global alignment score by performing pairwise global alignment on a base sequence which is of an up to three base length and includes the 3' terminal of the base sequence of the above-described primer candidate; ($f_1$) a first step of second stage selection of performing second stage selection of the base sequence of the above-described primer candidate based on the global alignment score obtained in the above-described ($e_1$) first global alignment step; ($g_1$) a first primer employment step of employing the base sequence of the primer candidate which has been selected in both of the above-described ($d_1$) first step of first stage selection and the above-described ($f_1$) first step of second stage selection as a base sequence of a primer for amplifying the above-described first target region; ($a_n$) an n-th target region selection step of selecting an n-th target region to be amplified through the polymerase chain reaction, from regions on a genome; ($b_n$) an n-th primer candidate base sequence generation step of generating at least one base sequence of a primer candidate for amplifying the above-described n-th target region based on each base sequence in vicinity regions at both ends of the above-described n-th target region on the genome; ($c_n$) an n-th local alignment step of obtaining a local alignment score by performing pairwise local alignment on the base sequence of the primer candidate for amplifying the above-described n-th target region and the base sequence of the primer which has already been employed, under a condition that partial sequences to be subjected to comparison include the 3' terminal of the base sequence of the above-described primer candidate and the 3' terminal of the above-described base sequence of the primer which has already been employed; ($d_n$) an n-th step of first stage selection of performing first stage selection of the base sequence of the primer candidate for amplifying the above-described n-th target region based on the local alignment score obtained in the above-described ($c_n$) n-th local alignment step; ($e_n$) an n-th global alignment step of obtaining a global alignment score by performing pairwise global alignment on base sequences which are of an up to three base length and include the 3' terminal of the base sequence of the primer candidate for amplifying the above-described n-th target region and the 3' terminal of the base sequence of the primer which has already been employed; ($f_n$) an n-th step of second stage selection of performing second stage selection of the base sequence of the primer candidate for amplifying the above-described n-th target region based on the global alignment score obtained in the above-described ($e_n$) n-th global alignment step; and ($g_n$) an n-th primer employment step of employing the base sequence of the primer candidate which has been selected in both of the above-described ($d_n$) n-th step of first stage selection and the above-described ($f_n$) n-th step of second stage selection as a base sequence of a primer for amplifying the above-described n-th target region.

Here, n is an integer of 2 or more, and each step from the above-described ($a_n$) n-th target region selection step to the above-described ($g_n$) n-th primer employment step regarding all target regions is repeated until base sequences of primers for amplifying the target regions are employed and until n reaches a number of target regions selected in the objective region selection step.

Here, both steps of the above-described ($c_1$) first local alignment step and the above-described ($d_1$) first step of first stage selection are performed before or after both steps of the above-described ($e_1$) first global alignment step and the above-described ($f_1$) first step of second stage selection, or performed in parallel with the above-described ($e_1$) first global alignment step and the above-described ($f_1$) first step of second stage selection, and both steps of the above-described ($c_n$) n-th local alignment step and the above-described ($d_n$) n-th step of first stage selection are performed before or after both steps of the above-described ($e_n$) n-th global alignment step and the above-described ($f_n$) n-th step of second stage selection, or performed in parallel with the above-described ($e_n$) n-th global alignment step and the above-described ($f_n$) n-th step of second stage selection.

Each step of the second embodiment of the method for designing a primer in the present invention will be described in detail.

($a_1$) First Target Region Selection Step

($a_1$) First Target Region Selection Step is shown in the block diagram of Fig. 1 as "(FIRST) TARGET REGION SELECTION STEP".

($a_1$) First Target Region Selection Step is the same as the above-described "(a) Target Region Selection Step" of the first embodiment except that one gene region is selected as a first target region from regions on a genome.

($b_1$) First Primer Candidate Base Sequence Generation Step

($b_1$) First Primer Candidate Base Sequence Generation Step is shown in the block diagram of Fig. 1 as "(FIRST) PRIMER CANDIDATE BASE SEQUENCE GENERATION STEP".

($b_1$) First Primer Candidate Base Sequence Generation Step is the same as "(b) Primer Candidate Base Sequence Generation Step" of the first embodiment of the designing method of the present invention except that a base sequence of a primer candidate for amplifying the first target region selected in the above-described ($a_1$) First Target Region Selection Step is generated.

(Specificity-Checking Step)

The specificity-checking step is the same as "Specificity-Checking Step" of the first embodiment of the designing method of the present invention. The present step is an arbitrary step, and may be performed or may not be performed.

($c_1$) First Local Alignment Step

($c_1$) First Local Alignment Step is shown in the block diagram of Fig. 1 as "(FIRST) LOCAL ALIGNMENT STEP".

($c_1$) First Local Alignment Step is the same as "(c) Local Alignment Step" of the first embodiment of the designing method of the present invention except that local alignment is performed on the base sequence of the primer candidate for amplifying the first target region generated in the above-described ($b_1$) First Primer Candidate Base Sequence Generation Step.

($d_1$) First Step of First Stage Selection

($d_1$) First Step of First Stage Selection is shown in the block diagram of Fig. 1 as "(FIRST) STEP OF FIRST STAGE SELECTION".

($d_1$) First Step of First Stage Selection is the same as "(d) First Stage Selection Step" of the first embodiment of the designing method of the present invention except that the selection is performed on the base sequence of the primer candidate for amplifying the first target region generated in the above-described ($b_1$) First Primer Candidate Base Sequence Generation Step based on the local alignment score obtained in the above-described ($c_1$) First Local Alignment Step.

(e₁) First Global Alignment Step

(e₁) First Global Alignment Step is shown in the block diagram of Fig. 1 as "(FIRST) GLOBAL ALIGNMENT STEP".

(e₁) First Global Alignment Step is the same as "(e) Global Alignment Step" of the first embodiment of the designing method of the present invention except that global alignment is performed on the base sequence of the primer candidate for amplifying the first target region generated in the above-described (b₁) First Primer Candidate Base Sequence Generation Step.

(f₁) First Step of Second Stage Selection

(f₁) First Step of Second Stage Selection is shown in the block diagram of Fig. 1 as "(FIRST) STEP OF SECOND STAGE SELECTION".

(f₁) First Step of Second Stage Selection is the same as "(f) Second Stage Selection Step" of the first embodiment of the designing method of the present invention except that the selection is performed on the base sequence of the primer candidate for amplifying the first target region generated in the above-described (b₁) First Primer Candidate Base Sequence Generation Step based on the global alignment score obtained in the above-described (e₁) First Global Alignment Step.

Similarly to the first embodiment of the designing method of the present invention, both steps of the above-described (c₁) First Local Alignment Step and the above-described (d₁) First Step of First Stage Selection may be performed before or after both steps of the above-described (e₁) First Global Alignment Step and the above-described (f₁) First Step of Second Stage Selection, or may be performed in parallel with both steps of the above-described (e₁) First Global Alignment Step and the above-described (f₁) First Step of Second Stage Selection.

In addition, similarly to the first embodiment, in order to reduce the amount of calculation, it is preferable to perform "(c₁) First Local Alignment Step" and "(d₁) First Step of First Stage Selection" in a combination which has passed the first step of second stage selection after first performing "(e₁) First Global Alignment Step" and "(f₁) First step of Second Stage Selection".

(Amplification Sequence Length-Checking Step)

Amplification Sequence Length-Checking Step is the same as "Amplification Sequence Length-Checking Step" in the first embodiment. The present step is an arbitrary step, and may be performed or may not be performed.

(g₁) First Primer Employment Step

(g₁) First Primer Employment Step is shown in the block diagram of Fig. 1 as "(FIRST) PRIMER EMPLOYMENT STEP".

(g₁) First Primer Employment Step is the same as "(g) Primer Employment Step" of the first embodiment of the designing method of the present invention except that the base sequence of the primer candidate for amplifying the first target region generated in the above-described (b₁) First Primer Candidate Base Sequence Generation Step is employed.

In the second embodiment of the present invention, a primer for amplifying the first target region is designed, and then, a primer for amplifying an n-th (n is an integer of 2 or more) target region is designed.

(aₙ) n-th Target Region Selection Step

(aₙ) n-th Target Region Selection Step is shown in the block diagram of Fig. 1 as "n-th TARGET REGION SELECTION STEP".

(aₙ) n-th Target Region Selection Step is the same as the above-described "(a) Target Region Selection Step" of the first embodiment except that one gene region is selected as an n-th target region from regions on a genome in which no target region has been selected up to an (n-1)th target region selection step.

The selection of the n-th target region can be simultaneously performed with the selection of an (n-1)th target region, or can be performed after the selection of the (n-1)th target region. Here, n is an integer of 2 or more.

(bₙ) n-th Primer Candidate Base Sequence Generation Step

(bₙ) n-th Primer Candidate Base Sequence Generation Step is shown in the block diagram of Fig. 1 as "n-th PRIMER CANDIDATE BASE SEQUENCE GENERATION STEP".

(bₙ) n-th Primer Candidate Base Sequence Generation Step is the same as "(b) Primer Candidate Base Sequence Generation Step" of the first embodiment of the designing method of the present invention except that a base sequence of a primer candidate for amplifying an n-th target region selected in the above-described $(a_n)$ n-th Target Region Selection Step is generated.

(Specificity-Checking Step)

Specificity-Checking Step is the same as "Specificity-Checking Step" of the first embodiment of the designing method of the present invention. The present step is an arbitrary step, and may be performed or may not be performed.

$(c_n)$ n-th Local Alignment Step

$(c_n)$ n-th Local Alignment Step is shown in the block diagram of Fig. 1 as "n-th LOCAL ALIGNMENT STEP".
$(c_n)$ n-th Local Alignment Step is the same as "(c) Local Alignment Step" of the first embodiment of the designing method of the present invention except that local alignment is performed on the base sequence of the primer candidate for amplifying the n-th target region generated in the above-described $(b_n)$ n-th Primer Candidate Base Sequence Generation Step and base sequences of primers which have already been employed.
Here, all the base sequences of the primers which have already been employed are base sequences which have been employed as base sequences of primers for amplifying target regions from the first target region to the (n-1)th target region (the same applies hereinafter).

$(d_n)$ n-th Step of First Stage Selection

$(d_n)$ n-th Step of First Stage Selection is shown in the block diagram of Fig. 1 as "n-th STEP OF FIRST STAGE SELECTION".
$(d_n)$ n-th Step of First Stage Selection is the same as "(d) First Stage Selection Step" of the first embodiment of the designing method of the present invention except that the selection is performed on the base sequence of the primer candidate for amplifying the n-th target region generated in the above-described $(b_n)$ n-th Primer Candidate Base Sequence Generation Step and the base sequences of the primers which have already been employed, based on the local alignment score obtained in the above-described $(c_n)$ n-th Local Alignment Step.

$(e_n)$ n-th Global Alignment Step

$(e_n)$ n-th Global Alignment Step is shown in the block diagram of Fig. 1 as "n-th GLOBAL ALIGNMENT STEP".
$(e_n)$ n-th Global Alignment Step is the same as "(e) Global Alignment Step" of the first embodiment of the designing method of the present invention except that global alignment is performed on the base sequence of the primer candidate for amplifying the n-th target region generated in the above-described $(b_n)$ n-th Primer Candidate Base Sequence Generation Step and the base sequences of the primers which have already been employed.

$(f_n)$ n-th Step of Second Stage Selection

$(f_n)$ n-th Step of Second Stage Selection is shown in the block diagram of Fig. 1 as "n-th Step of Second Stage Selection".
$(f_n)$ n-th Step of Second Stage Selection is the same as "(f) Second Stage Selection Step" of the first embodiment of the designing method of the present invention except that the selection is performed on the base sequence of the primer candidate for amplifying the n-th target region generated in the above-described $(b_n)$ n-th Primer Candidate Base Sequence Generation Step based on the global alignment score obtained in the above-described $(e_n)$ n-th Global Alignment Step and the base sequences of the primers which have already been employed.
Similarly to the first embodiment of the designing method of the present invention, both steps of the above-described $(c_n)$ n-th Local Alignment Step and the above-described $(d_n)$ n-th Step of First Stage Selection may be performed before or after both steps of the above-described $(e_n)$ n-th Global Alignment Step and the above-described $(f_n)$ n-th Step of Second Stage Selection, or may be performed in parallel with both steps of the above-described $(e_n)$ n-th Global Alignment Step and the above-described $(f_n)$ n-th Step of Second Stage Selection.
In addition, in order to reduce the amount of calculation, it is preferable to perform both steps of the above-described $(c_n)$ n-th Local Alignment Step and the above-described $(d_n)$ n-th Stage Selection Step in a combination which has passed the above-described $(f_n)$ n-th Step of Second Stage Selection after performing both steps of the above-described $(e_n)$ n-th Global Alignment Step and the above-described $(f_n)$ n-th Step of Second Stage Selection" first. Particularly, as the number of target regions and the number of base sequences of primer candidates are increased, the effect of reducing the amount of calculation is increased, and it is possible to speed up the overall processing.

(Amplification Sequence Length-Checking Step)

Amplification Sequence Length-Checking Step is the same as "Amplification Sequence Length-Checking Step" of the first embodiment of the designing method of the present invention. The present step is an arbitrary step, and may be performed or may not be performed.

(g_n) n-th Primer Employment Step

(g_n) n-th Primer Employment Step is shown in the block diagram of Fig. 1 as "n-th PRIMER EMPLOYMENT STEP".

(g_n) n-th Primer Employment Step is the same as "(g) Primer Employment Step" of the first embodiment of the designing method of the present invention except that the base sequence of the primer candidate for amplifying the n-th target region generated in the above-described (b_n) n-th Primer Candidate Base Sequence Generation Step is employed.

[Primer Set Used for Polymerase Chain Reaction]

The primer set described herein used for a polymerase chain reaction of the present invention is a set of primers designed through the above-described method for designing a primer used for a polymerase chain reaction.

That is, a local alignment score obtained by performing pairwise local alignment on a base sequence of each primer under a condition that a partial sequence to be subjected to comparison includes the 3' terminal of the above-described base sequence is less than a first threshold value, and a global alignment score obtained by performing pairwise global alignment on a base sequence which has a length of up to three bases and the 3' terminal of the base sequence of each primer is less than a second threshold value.

It is possible to set the global alignment score obtained by performing pairwise global alignment on a base sequence which has a predetermined number of bases and includes the 3' terminal of a base sequence of each primer to be less than the second threshold value by setting a base sequence with several bases from the 3' terminal of a primer as an identical base sequence.

It is possible to perform SNP determination and/or SNV determination on a trace amount of DNA using the present invention. Furthermore, by setting a target region to be an arbitrary position within a specific chromosome, it is possible to know the quantitative ratio for each specific chromosome. Accordingly, it is possible to perform a genome abnormality test, such as new prenatal diagnosis, using single cells or a trace amount of DNA.

Hereinafter, the present invention will be described in more detail using Examples, but is not limited to these Examples.

[Examples]

[Example 1]

(1) Selection of Target Region

SNP positions shown in Table 8 were selected as target regions. An SNP ID is an identification number used in a single nucleotide polymorphism database (dbSNP) managed by National Center for Biotechnology Information (NCBI).

[Table 8]

Table 8

| SNP ID | Chromosome Number | Coordinate | Allele |
|---|---|---|---|
| rs7981616 | 13 | 25265103 | A/G |
| rs2230233 | 18 | 29104698 | C/T |
| rs2073370 | 21 | 35260481 | T/C |
| rs2379206 | X | 6995315 | C/T |

(2) Generation of Primer Candidate Base Sequence

Primer candidate base sequences (SEQ ID No: 1 to SEQ ID No: 8) shown in Table 9 were respectively generated with respect to the selected target regions. Here, a "forward primer" refers to a primer generated based on a base sequence of the 5' side region (a region on a side where a coordinate is small) of an SNP position and a "reverse primer" refers to a primer generated based on a base sequence (a base sequence of a complementary chain of chromosome DNA) of the 3' side region (a region on a side where a coordinate is large) of an SNP position.

[Table 9]

Table 9

| SEQ ID No | Target region | Primer candidate base sequence | Amplification start / finish position | Distance between primers |
| --- | --- | --- | --- | --- |
| | SNP ID | Upper part: forward primer (5' → 3') | Upper part: amplification start position | |
| | | Lower part: reverse primer (5' → 3') | Lower part: amplification finish position | |
| 1 | rs7981616 | GCTTGGCCTTGGGAATGTGG | 25264999 | 180 |
| 2 | | GGCAATATGGCCAATGATGG | 25265178 | |
| 3 | rs2230233 | TTTGCAGCTTGAAGGGATGG | 29104659 | 161 |
| 4 | | GAGCATCTGTTTCTATGTGG | 29104819 | |
| 5 | rs2073370 | GCCTCGAAGAGAGGGAATGG | 35260401 | 171 |
| 6 | | GACCACAATCTCTCCCGTGG | 35260571 | |
| 7 | rs2379206 | AGGAAGATGTCCGGGTCTGG | 6995304 | 170 |
| 8 | | ATCCACCTGCGGAAACATGG | 6995473 | |

(3) Evaluation of Dimer Formability

In order to exclude a primer candidate base sequence which easily formed a primer dimer, evaluation of the dimer formability was performed using a local alignment score. Then, the dimer formability was further evaluated on a primer candidate base sequence which had been evaluated that the dimer formability was low, using a global alignment score, and a combination of primer candidate base sequences having low dimer formability was obtained.

a) Evaluation Using Local Alignment Score

Pairwise local alignment was performed on all pairs (28 pairs) of the primer candidate base sequences represented by SEQ ID No: 1 to SEQ ID No: 8 under the condition (constraint condition) that a partial base sequence to be subjected to comparison includes the 3' terminal of a primer candidate base sequence. The local alignment was set such that an alignment score became a maximum under the above-described constraint condition using the scoring system shown in Table 10. In Table 10, "-" represents a gap (indel: insertion/deletion).
A threshold value of the local alignment score was set to 3, and pairs of which the local alignment score was less than 3 were passed.

[Table 10]

Table 10

|   | A | T | G | C | - |
|---|---|---|---|---|---|
| A | -1 | +1 | -1 | -1 | -3 |
| T | +1 | -1 | -1 | -1 | -3 |
| G | -1 | -1 | -1 | +1 | -3 |
| C | -1 | -1 | +1 | -1 | -3 |
| - | -3 | -3 | -3 | -3 |   |

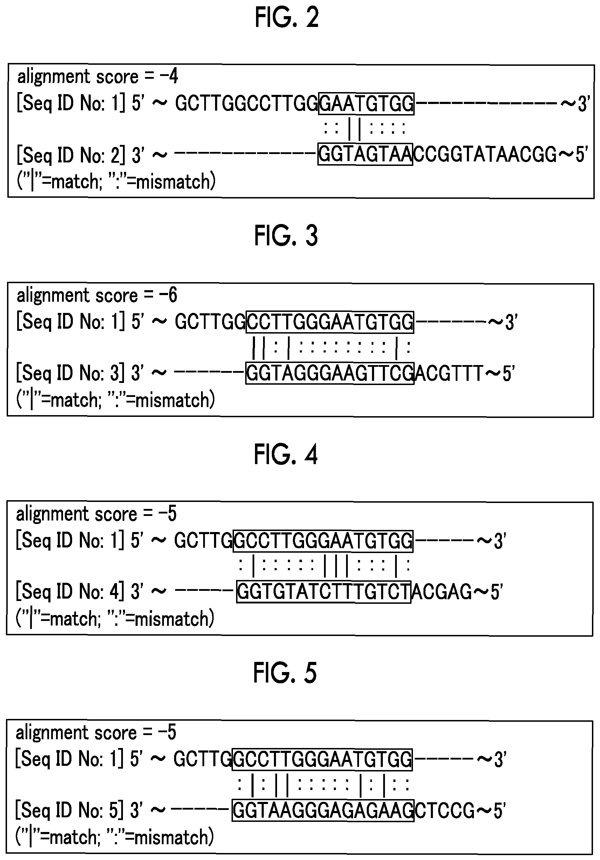

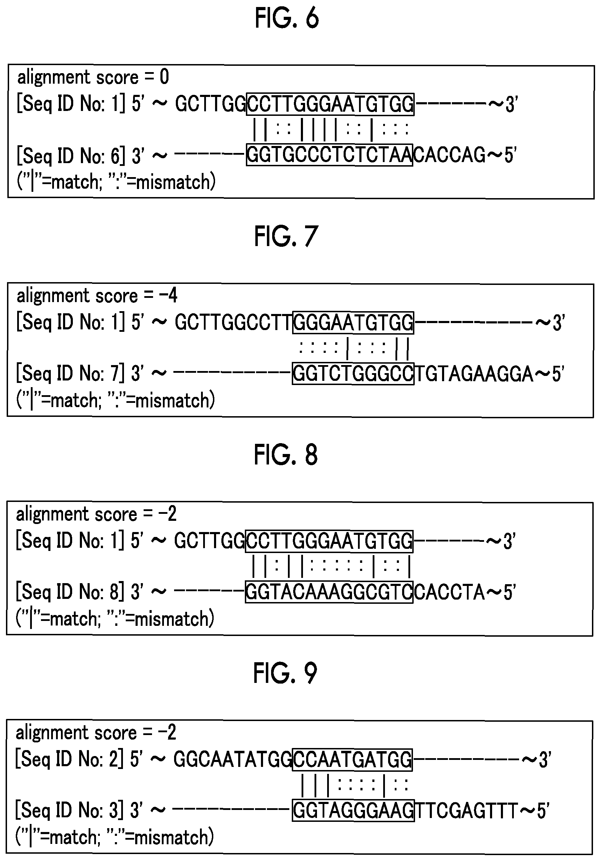

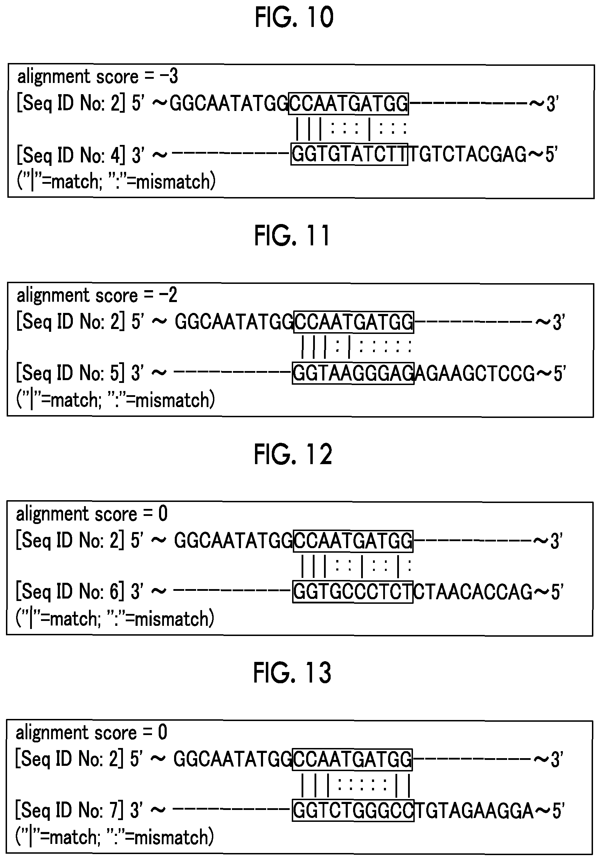

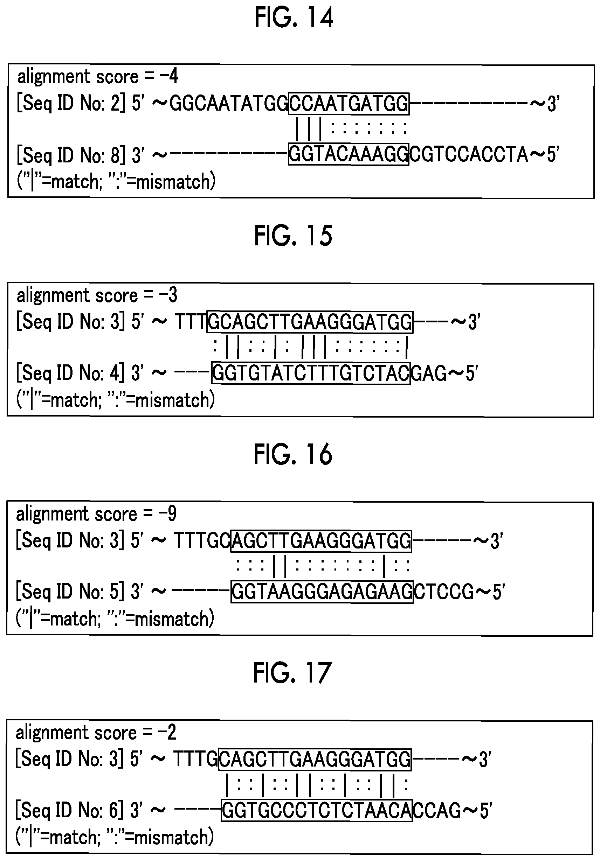

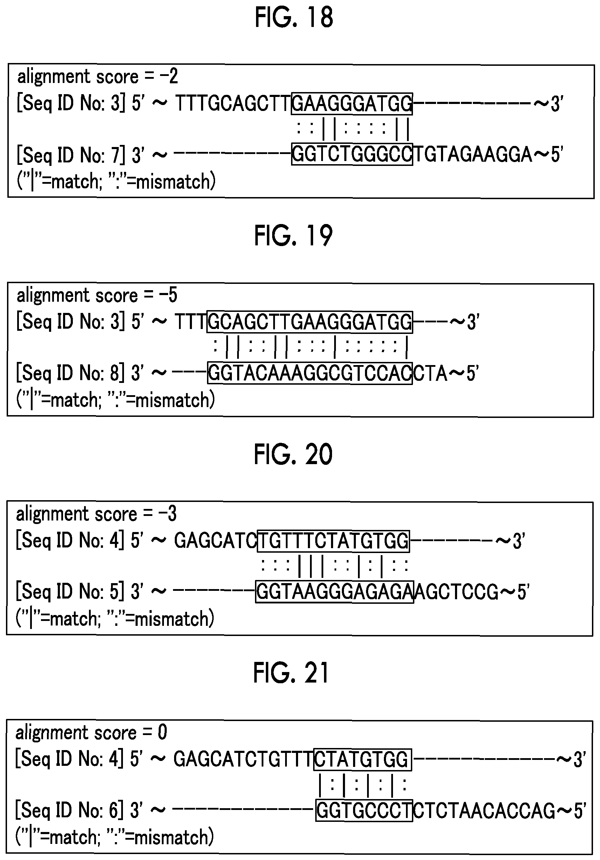

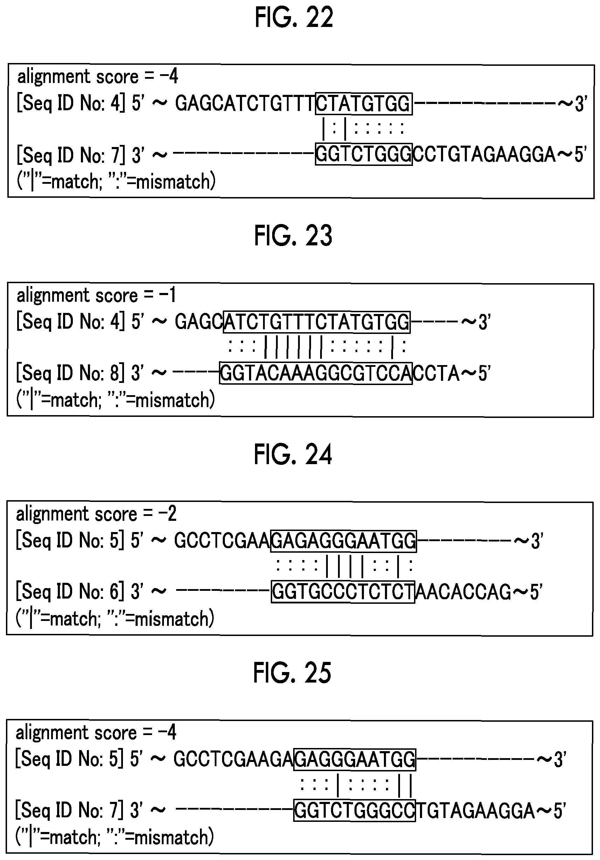

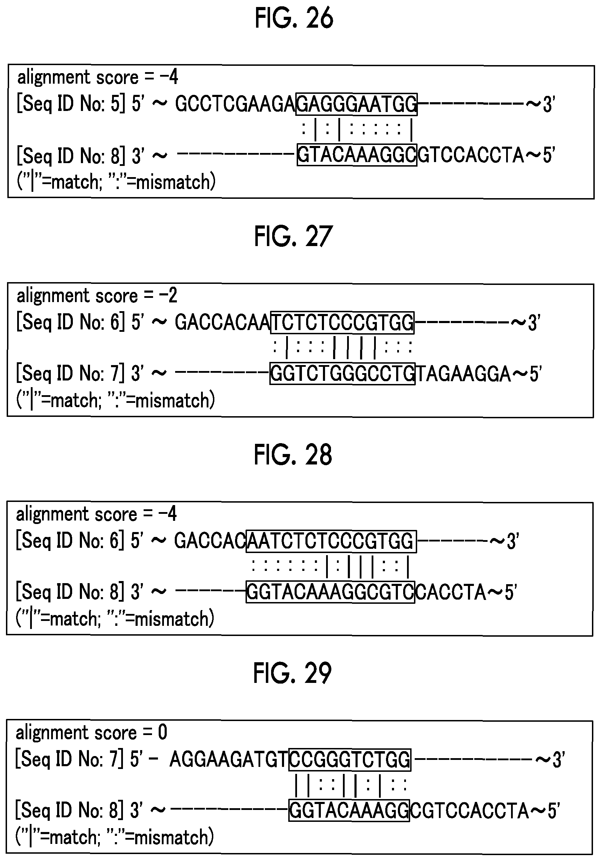

The obtained kinds of alignment are shown in Fig. 2 to Fig. 29. Local alignment scores for all of the combinations were less than "3", and therefore, it was evaluated that all of the combinations had low dimer formability.

b) Evaluation Using Global Alignment Score

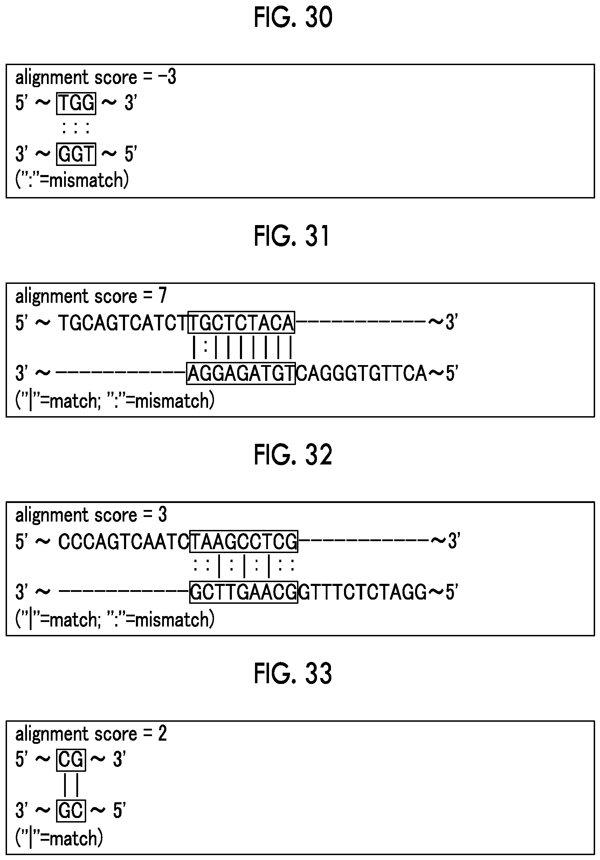

Three bases were extracted from the 3' terminal of each primer candidate base sequence and global alignment was performed on base sequences (all are 5'-TGG-3') each consisting of the three bases regarding all of the pairs (28 pairs) of the primer candidate base sequences represented by SEQ ID No: 1 to SEQ ID No: 8. The global alignment was set such that an alignment score became a maximum using a scoring matrix shown in Table 10. The threshold value of the score was set to 3, and pairs of which the global alignment score was less than 3 were passed. In Table 10, "-" represents a gap (indel: insertion/deletion).
The obtained alignment was shown in Fig. 30. Global alignment scores for all of the pairs were less than "3", and therefore, it was evaluated that all of the pairs had low dimer formability.

(4) Evaluation of Distance between Primers

From the combinations of the primer candidate base sequences which had low dimer formability and which were obtained through the above-described "(3) Evaluation of Dimer Formability", the distance between primers (the length from an amplification start position to an amplification end position) was calculated for each of the pairs (a pair of SEQ ID No: 1 and SEQ ID No: 2, a pair of SEQ ID No: 3 and SEQ ID No: 4, a pair of SEQ ID No: 5 and SEQ ID No: 6, and a pair of SEQ ID No: 7 and SEQ ID No: 8) of the primer candidates for subjecting each target region to PCR amplification.
Pairs of which the distance between primers was within a range of 160 to 180 bases were passed.
As shown in Table 9, the distance between primers of each pair of all of the primer candidates was within a range of 160 to 180 bases.

(5) Confirmation of Primer Set

Sets of primers (primer sets) consisting of the base sequences represented by SEQ ID No: 1 to SEQ ID No: 8 were obtained as primer sets for amplifying four SNP positions shown in Table 1 through multiplex PCR.
These primer sets can selectively amplify a target region efficiently at the same time through multiplex PCR even in a case where the primer dimer formability was low and an extremely small amount of genomic DNA extracted from a single cell was set as template DNA.

[Comparative Example 1]

(1) Generation of Primer Candidate Base Sequence

A pair of a base sequence represented by SEQ ID No: 9 and a base sequence represented by SEQ ID No: 10 were generated as primer candidate base sequences.

(2) Evaluation of Dimer Formability

A threshold value of the local alignment score was set to "3". Pairs of which the local alignment score was greater than or equal to "3" were regarded to have high dimer formability. The pairs were excluded from the subject of evaluation of the dimer formability to be further performed.

Local alignment was performed on the pair of the base sequences represented by SEQ ID No: 9 and SEQ ID No: 10 in the same manner as in Example 1.

The obtained alignment is shown in Fig. 31. The local alignment score of the pair of the base sequences represented by SEQ ID No: 9 and SEQ ID No: 10 was "7" being greater than or equal to "3" which was the threshold value. Therefore, this pair was excluded from the subject of evaluation of the dimer formability since this pair had high dimer formability.

A primer dimer is formed in a case of actually performing a polymerase chain reaction (PCR) using the primer represented by the base sequence of SEQ ID No: 9 and the primer represented by the base sequence of SEQ ID No: 10. It is possible to prevent a dimer obtained from alignment with such a high score, through the first stage selection step.

[Table 11]

Table 11

| | | SEQ ID No | Primer base sequence (5' → 3') |
|---|---|---|---|
| Comparative Example | 1 | 9 | TGCAGTCATCTTGCTCTACA |
| | | 10 | ACTTGTGGGACTGTAGAGGA |
| | 2 | 11 | CCCAGTCAATCTAAGCCTCG |
| | | 12 | GGATCTCTTTGGCAAGTTCG |

[Comparative Example 2]

(1) Generation of Primer Candidate Base Sequence

A pair of a base sequence represented by SEQ ID No: 11 and a base sequence represented by SEQ ID No: 12 were generated as primer candidate base sequences.

(2) Evaluation of Dimer Formability

a) Evaluation Using Local Alignment Score

A threshold value of the local alignment score was set to "3". Pairs of which the local alignment score was less than "3" were passed, and the evaluation of the dimer formability was further performed.

Local alignment was performed on a pair of the base sequences represented by SEQ ID No: 11 and SEQ ID No: 12 in the same manner as in Example 1.

The obtained alignment is shown in Fig. 32. The local alignment score of the pair of the base sequences represented by SEQ ID No: 11 and SEQ ID No: 12 was "-4" being less than "3" which was the threshold value. Therefore, evaluation of the dimer formability was performed on this pair.

b) Evaluation Using Global Alignment Score

A threshold value of the global alignment score was set to "2". Pairs of which the global alignment score was greater than or equal to "2" were regarded to have high dimer formability. The pairs were excluded from the subject of evaluation of the primer to be further performed.

Global alignment was performed on the pair of the base sequences represented by SEQ ID No: 11 and SEQ ID No: 12 in the same manner as in Example 1.

The obtained alignment is shown in Fig. 33. The global alignment score of two bases at the 3' terminal of the pair of the base sequences represented by SEQ ID No: 11 and SEQ ID No: 12 was "2" being greater than or equal to "2" which was the threshold value. Therefore, this pair was excluded from the subject of evaluation of the dimer formability since this pair had high dimer formability.

In this manner, since a primer dimer is formed with only several bases at the 3' terminal of a primer, the alignment score is low from the viewpoint of the entire sequence by performing the second stage selection step in addition to the first stage selection step. However, it is possible to prevent the dimer formation through annealing of only the several bases of the 3' terminal.

The method of the present invention can provide a primer set which can selectively amplify an objective region efficiently even in a case where the number of gene regions to be amplified is comparatively small or large and can be applied to applications which include genetic diagnostic applications and in which various PCR methods are used since the present invention is useful in PCR, such as polymerase chain reaction (PCR) amplification from a single cell, in a case where the trace amount of genomic deoxyribonucleic acid (DNA) is used as template DNA.

**Claims**

1. A method for designing a primer used for a polymerase chain reaction, the method comprising:

a target region selection step of selecting a target region to be amplified through the polymerase chain reaction, from regions on a genome;
a primer candidate base sequence generation step of generating at least one base sequence of a primer candidate for amplifying the target region based on each base sequence in vicinity regions at both ends of the target region on the genome;
a local alignment step of obtaining a local alignment score by performing pairwise local alignment on the base sequence of the primer candidate under a condition that a partial sequence to be subjected to comparison includes the 3' terminal of the base sequence of the primer candidate;
a first stage selection step of performing first stage selection of the base sequence of the primer candidate based on the local alignment score obtained in the local alignment step;
a global alignment step of obtaining a global alignment score by performing pairwise global alignment on a base sequence which is of an up to three base length and includes the 3' terminal of the base sequence of the primer candidate;
a second stage selection step of performing second stage selection of a base sequence of the primer candidate based on the global alignment score obtained in the global alignment step; and

a primer employment step of employing the base sequence of the primer candidate which has been selected in both of the first stage selection step and the second stage selection step as a base sequence of a primer for amplifying the target region,

wherein both steps of the local alignment step and the first stage selection step are performed before or after both steps of the global alignment step and the second stage selection step, or performed in parallel with both steps of the global alignment step and the second stage selection step.

2.  The method for designing a primer used for a polymerase chain reaction according to claim 1, the method further comprising:

a first target region selection step of selecting a first target region to be amplified through the polymerase chain reaction, from regions on a genome;

a first primer candidate base sequence generation step of generating at least one base sequence of a primer candidate for amplifying the first target region based on each base sequence in vicinity regions at both ends of the first target region on the genome;

a first local alignment step of obtaining a local alignment score by performing pairwise local alignment on the base sequence of the primer candidate under a condition that a partial sequence to be subjected to comparison includes the 3' terminal of the base sequence of the primer candidate;

a first step of first stage selection of performing first stage selection of the base sequence of the primer candidate based on the local alignment score obtained in the local alignment step;

a first global alignment step of obtaining a global alignment score by performing pairwise global alignment on a base sequence which is of an up to three base length and includes the 3' terminal of the base sequence of the primer candidate;

a first step of second stage selection of performing second stage selection of the base sequence of the primer candidate based on the global alignment score obtained in the global alignment step;

a first primer employment step of employing the base sequence of the primer candidate which has been selected in both of the first step of first stage selection and the first step of second stage selection as a base sequence of a primer for amplifying the first target region;

a second target region selection step of selecting a second target region to be amplified through the polymerase chain reaction, from regions on a genome;

a second primer candidate base sequence generation step of generating at least one base sequence of a primer candidate for amplifying the second target region based on each base sequence in vicinity regions at both ends of the second target region on the genome;

a second local alignment step of obtaining a local alignment score by performing pairwise local alignment on the base sequence of the primer candidate for amplifying the second target region and the base sequence of the primer which has already been employed, under a condition that partial sequences to be subjected to comparison include the 3' terminal of the base sequence of the primer candidate and the 3' terminal of the base sequence of the primer which has already been employed;

a second step of first stage selection of performing first stage selection of the base sequence of the primer candidate for amplifying the second target region based on the local alignment score;

a second global alignment step of obtaining a global alignment score by performing pairwise global alignment on base sequences which are of an up to three base length and include the 3' terminal of the base sequence of the primer candidate for amplifying the second target region and the 3' terminal of the base sequence of the primer which has already been employed;

a second step of second stage selection of performing second stage selection of the base sequence of the primer candidate for amplifying the second target region based on the global alignment score; and

a second primer employment step of employing the base sequence of the primer candidate which has been selected in both of the second step of first stage selection and the second step of second stage selection as a base sequence of a primer for amplifying the second target region,

wherein both steps of the first local alignment step and the first step of first stage selection are performed before or after both steps of the first global alignment step and the first step of second stage selection, or performed in parallel with both steps of the first global alignment step and the first step of second stage selection,

wherein both steps of the second local alignment step and the second step of first stage selection are performed before or after both steps of the second global alignment step and the second step of second stage selection, or performed in parallel with both steps of the second global alignment step and the second step of second stage selection, and

wherein, in a case where there are three or more of the target regions, the steps from the second target region selection step to the second primer employment step are repeated with respect to all of the target regions until a base sequence of a primer for amplifying each target region is employed.

**Patentansprüche**

1. Verfahren zum Entwerfen eines Primers, der für eine Polymerasekettenreaktion verwendet wird, wobei das Verfahren aufweist:

   einen Zielregion-Auswahlschritt des Auswählens einer Zielregion, die durch die Polymerasekettenreaktion amplifiziert werden soll, aus Regionen auf einem Genom;
   einen Primerkandidaten-Basensequenz-Erzeugungsschritt des Erzeugens mindestens einer Basensequenz eines Primerkandidaten zum Amplifizieren der Zielregion auf der Basis jeder Basensequenz in Nachbarregionen an beiden Enden der Zielregion auf dem Genom;
   einen lokalen Alignmentschritt des Erhaltens eines lokalen Alignment-Scores durch Ausführen eines paarweisen lokalen Alignments an der Basensequenz des Primerkandidaten unter einer Bedingung, dass eine Teilsequenz, die einem Vergleich unterzogen werden soll, das 3'-Ende der Basensequenz des Primerkandidaten enthält;
   einen Erstphasen-Auswahlschritt des Durchführens einer Erstphasen-Auswahl der Basensequenz des Primerkandidaten auf der Basis des lokalen Alignment-Scores, der in dem lokalen Alignmentschritt erhalten wurde;
   einen globalen Alignmentschritt des Erhaltens eines globalen Alignment-Scores durch Ausführen eines paarweisen globalen Alignments an einer Basensequenz, die eine Länge von bis zu drei Basen hat und das 3'-Ende der Basensequenz des Primerkandidaten enthält;
   einen Zweitphasen-Auswahlschritt des Durchführens einer Zweitphasen-Auswahl einer Basensequenz des Primerkandidaten auf der Basis des globalen Alignment-Scores, der in dem globalen Alignmentschritt erhalten wurde; und
   einen Primeranwendungsschritt des Anwendens der Basensequenz des Primerkandidaten, die sowohl in dem Erstphasen-Auswahlschritt als auch in dem Zweitphasen-Auswahlschritt ausgewählt wurde, als Basensequenz eines Primers zum Amplifizieren der Zielregion,
   wobei sowohl der lokale Alignmentschritt als auch der Erstphasen-Auswahlschritt vor oder nach sowohl dem globalen Alignmentschritt als auch dem Zweitphasen-Auswahlschritt durchgeführt werden oder parallel mit sowohl dem globalen Alignmentschritt als auch dem Zweitphasen-Auswahlschritt durchgeführt werden.

2. Verfahren zum Entwerfen eines Primers, der für eine Polymerasekettenreaktion verwendet wird, nach Anspruch 1, wobei das Verfahren außerdem aufweist:

   einen ersten Zielregion-Auswahlschritt des Auswählens einer ersten Zielregion, die durch die Polymerasekettenreaktion amplifiziert werden soll, aus Regionen auf einem Genom;
   einen ersten Primerkandidaten-Basensequenz-Erzeugungsschritt des Erzeugens mindestens einer Basensequenz eines Primerkandidaten zum Amplifizieren der ersten Zielregion auf der Basis jeder Basensequenz in Nachbarregionen an beiden Enden der ersten Zielregion an dem Genom;
   einen ersten lokalen Alignmentschritt des Erhaltens eines lokalen Alignment-Scores durch Ausführen eines paarweisen lokalen Alignments an der Basensequenz des Primerkandidaten unter der Bedingung, dass eine zu vergleichende Teilsequenz das 3'-Ende der Basensequenz des Primerkandidaten enthält;
   einen ersten Erstphasen-Auswahlschritt des Durchführens einer Erstphasen-Auswahl der Basensequenz des Primerkandidaten auf der Basis des lokalen Alignment-Scores, der in dem lokalen Alignmentschritt erhalten wurde;
   einen ersten globalen Alignmentschritt des Erhaltens eines globalen Alignment-Scores durch Ausführen eines paarweisen globalen Alignments an einer Basensequenz, die eine Länge von bis zu drei Basen hat und das 3'-Ende der Basensequenz des Primerkandidaten enthält;
   einen ersten Zweitphasen-Auswahlschritt des Durchführens einer Zweitphasen-Auswahl der Basensequenz des Primerkandidaten auf der Basis des globalen Alignment-Scores, der in dem globalen Alignmentschritt erhalten wurde;
   einen ersten Primeranwendungsschritt des Anwendens der Basissequenz des Primerkandidaten, die sowohl in dem ersten Erstphasen-Auswahlschritt als auch in dem ersten Zweitphasen-Auswahlschritt ausgewählt wurde, als eine Basensequenz eines Primers zum Amplifizieren der ersten Zielregion;
   einen zweiten Zielregion-Auswahlschritt des Auswählens einer zweiten Zielregion, die durch die Polymerasekettenreaktion amplifiziert werden soll, aus Regionen auf einem Genom;
   einen zweiten Primerkandidaten-Basensequenz-Erzeugungsschritt des Erzeugens mindestens einer Basensequenz eines Primerkandidaten zum Amplifizieren der zweiten Zielregion auf der Basis jeder Basensequenz in Nachbarregionen an beiden Enden der zweiten Zielregion auf dem Genom;

einen zweiten lokalen Alignmentschritt des Erhaltens eines lokalen Alignment-Scores durch Ausführen eines paarweisen lokalen Alignments an der Basensequenz des Primerkandidaten zum Amplifizieren der zweiten Zielregion und der Basensequenz des Primers, der bereits angewendet wurde, unter der Bedingung, dass zu vergleichende Teilsequenzen das 3'-Ende der Basensequenz des Primerkandidaten und das 3'-Ende der Basensequenz des Primers, der bereits angewendet wurde, enthalten;

einen zweiten Erstphasen-Auswahlschritt des Durchführens einer Erstphasen-Auswahl der Basensequenz des Primerkandidaten zum Amplifizieren der zweiten Zielregion auf der Basis des lokalen Alignment-Scores;

einen zweiten globalen Alignmentschritt des Erhaltens eines globalen Alignment-Scores durch Ausführen eines paarweisen globalen Alignments an Basensequenzen, die eine Länge von bis zu drei Basen haben und das 3'-Ende der Basensequenz des Primerkandidaten zum Amplifizieren der zweiten Zielregion und das 3'-Ende der Basensequenz des Primers, der bereits verwendet wurde, enthalten;

einen zweiten Zweitphasen-Auswahlschritt des Durchführens einer Zweitphasen-Auswahl der Basensequenz des Primerkandidaten zum Amplifizieren der zweiten Zielregion auf der Basis des globalen Alignment-Scores; und

einen zweiten Primeranwendungsschritt des Anwendens der Basensequenz des Primerkandidaten, die sowohl in dem zweiten Erstphasen-Auswahlschritt als auch in dem zweiten Zweitphasen-Auswahlschritt ausgewählt wurde, als eine Basensequenz eines Primers zum Amplifizieren der zweiten Zielregion,

wobei sowohl der erste lokale Alignmentschritt als auch der erste Erstphasen-Auswahlschritt vor oder nach sowohl dem ersten globalen Alignmentschritt als auch dem ersten Zweitphasen-Auswahlschritt durchgeführt werden, oder parallel mit sowohl dem ersten globalen Alignmentschritt als auch dem ersten Zweitphasen-Auswahlschritt durchgeführt werden,

wobei sowohl der zweite lokale Alignmentschritt als auch der zweite Erstphasen-Auswahlschritt vor oder nach sowohl dem zweiten globalen Alignmentschritt als auch dem zweiten Zweitphasen-Auswahlschritt durchgeführt werden, oder parallel sowohl mit dem zweiten globalen Alignmentschritt als auch dem zweiten Zweitphasen-Auswahlschritt durchgeführt werden, und

wobei in einem Fall, in dem es drei oder mehr Zielregionen gibt, die Schritte vom zweiten Zielregion-Auswahlschritt bis zum zweiten Primeranwendungsschritt bezüglich aller Zielregionen wiederholt werden bis eine Basensequenz eines Primers zum Amplifizieren jeder Zielregion verwendet wird.


## Revendications

1. Procédé destiné à concevoir une amorce utilisée pour une amplification en chaîne par polymérase, le procédé comprenant les étapes suivantes :

   une étape de sélection de région cible pour sélectionner une région cible à amplifier par le biais de l'amplification en chaîne par polymérase, parmi des régions sur un génome ;

   une étape de génération de séquence de bases d'amorce candidate pour générer au moins une séquence de bases d'une amorce candidate pour amplifier la région cible sur la base de chaque séquence de bases dans des régions avoisinantes aux deux bouts de la région cible sur le génome ;

   une étape d'alignement local pour obtenir un score d'alignement local en réalisant un alignement local par paires sur la séquence de bases de l'amorce candidate à condition qu'une séquence partielle à comparer inclue l'extrémité 3' de la séquence de bases de l'amorce candidate ;

   une étape de sélection de premier stade pour réaliser une sélection de premier stade de la séquence de bases de l'amorce candidate sur la base du score d'alignement local obtenu lors de l'étape d'alignement local ;

   une étape d'alignement global pour obtenir un score d'alignement global en réalisant un alignement global par paires sur une séquence de bases, laquelle présente une longueur allant jusqu'à trois bases et inclut l'extrémité 3' de la séquence de bases de l'amorce candidate ;

   une étape de sélection de second stade pour réaliser une sélection de second stade d'une séquence de bases de l'amorce candidate sur la base du score d'alignement global obtenu lors de l'étape d'alignement global ;

   une étape d'emploi d'amorce pour employer la séquence de bases de l'amorce candidate, laquelle a été sélectionnée à la fois lors de l'étape de sélection de premier stade et de l'étape de sélection de second stade comme séquence de bases d'une amorce pour amplifier la région cible, et

   dans lequel les deux étapes de l'étape d'alignement local et de l'étape de sélection de premier stade sont réalisées avant ou après les deux étapes de l'étape d'alignement global et de l'étape de sélection de second stade, ou sont réalisées parallèlement aux deux étapes de l'étape d'alignement global et de l'étape de sélection de second stade.

2. Procédé destiné à concevoir une amorce utilisée pour une amplification en chaîne par polymérase selon la revendication 1, le procédé comprenant en outre les étapes suivantes :

une étape de sélection de première région cible pour sélectionner une première région cible à amplifier par le biais de l'amplification en chaîne par polymérase, parmi des régions sur un génome ;
une première étape de génération de séquence de bases d'amorce candidate pour générer au moins une séquence de bases d'une amorce candidate pour amplifier la première région cible sur la base de chaque séquence de bases dans des régions avoisinantes aux deux bouts de la première région cible sur le génome ;
une première étape d'alignement local pour obtenir un score d'alignement local en réalisant un alignement local par paires sur la séquence de bases de l'amorce candidate à condition qu'une séquence partielle à comparer inclue l'extrémité 3' de la séquence de bases de l'amorce candidate ;
une première étape de sélection de premier stade pour réaliser une sélection de premier stade de la séquence de bases de l'amorce candidate sur la base du score d'alignement local obtenu lors de l'étape d'alignement local ;
une première étape d'alignement global pour obtenir un score d'alignement global en réalisant un alignement global par paires sur la séquence de bases, laquelle présente une longueur allant jusqu'à trois bases et inclut l'extrémité 3' de la séquence de bases de l'amorce candidate ;
une première étape de sélection de second stade pour réaliser une sélection de second stade de la séquence de bases de l'amorce candidate sur la base du score d'alignement global obtenu lors de l'étape d'alignement global ;
une première étape d'emploi d'amorce pour employer la séquence de bases de l'amorce candidate, laquelle a été sélectionnée à la fois lors de la première étape de sélection de premier stade et de la première étape de sélection de second stade comme séquence de bases d'une amorce pour amplifier la première région cible ;
une étape de sélection de seconde région cible pour sélectionner une seconde région cible à amplifier par le biais de l'amplification en chaîne par polymérase, parmi des régions sur un génome ;
une seconde étape de génération de séquence de bases d'amorce candidate pour générer au moins une séquence de bases d'une amorce candidate pour amplifier la seconde région cible sur la base de chaque séquence de bases dans des régions avoisinantes aux deux bouts de la seconde région cible sur le génome ;
une seconde étape d'alignement local pour obtenir un score d'alignement local en réalisant un alignement local par paires sur la séquence de bases de l'amorce candidate pour amplifier la seconde région cible et la séquence de bases de l'amorce qui a déjà été employée, à condition que des séquences partielles à comparer incluent l'extrémité 3' de la séquence de bases de l'amorce candidate et l'extrémité 3' de la séquence de bases de l'amorce qui a déjà été employée ;
une seconde étape de sélection de premier stade pour réaliser une sélection de premier stade de la séquence de bases de l'amorce candidate pour amplifier la seconde région cible sur la base du score d'alignement local ;
une seconde étape d'alignement global pour obtenir un score d'alignement global en réalisant un alignement global par paires sur la séquence de bases, laquelle présente une longueur allant jusqu'à trois bases et inclut l'extrémité 3' de la séquence de bases de l'amorce candidate pour amplifier la seconde région cible et l'extrémité 3' de la séquence de bases de l'amorce qui a déjà été employée ;
une seconde étape de sélection de second stade pour réaliser une sélection de second stade de la séquence de bases de l'amorce candidate pour amplifier la seconde région cible sur la base du score d'alignement global, et
une seconde étape d'emploi d'amorce pour employer la séquence de bases de l'amorce candidate, laquelle a été sélectionnée à la fois lors de la seconde étape de sélection de premier stade et de la seconde étape de sélection de second stade comme séquence de bases d'une amorce pour amplifier la seconde région cible,
dans lequel les deux étapes de la première étape d'alignement local et de la première étape de sélection de premier stade sont réalisées avant ou après les deux étapes de la première étape d'alignement global et de la première étape de sélection de second stade, ou sont réalisées parallèlement aux deux étapes de la première étape d'alignement global et de la première étape de sélection de second stade ;
dans lequel les deux étapes de la seconde étape d'alignement local et de la seconde étape de sélection de premier stade sont réalisées avant ou après les deux étapes de la seconde étape d'alignement global et de la seconde étape de sélection de second stade, ou sont réalisées parallèlement aux deux étapes de la seconde étape d'alignement global et de la seconde étape de sélection de second stade, et
dans lequel dans un cas où il existe trois ou plus régions cibles, les étapes allant de l'étape de sélection de seconde région cible à la seconde étape d'emploi d'amorce sont répétées par rapport à la totalité des régions cibles jusqu'à ce qu'une séquence de bases d'une amorce pour amplifier chaque région cible soit employée.

# FIG. 1

```
┌─────────────────────────────────────────────┐
│      (FIRST) TARGET REGION SELECTION STEP     │
└─────────────────────────────────────────────┘
                      ↓
┌─────────────────────────────────────────────┐
│ (FIRST) PRIMER CANDIDATE BASE SEQUENCE GENERATION STEP │
└─────────────────────────────────────────────┘
                      ↓
         ┌──────────────────────────┐
         │   SPECIFICITY-CHECKING STEP   │
         └──────────────────────────┘
```

| (FIRST) LOCAL ALIGNMENT STEP | (FIRST) GLOBAL ALIGNMENT STEP |
|---|---|
| (FIRST) STEP OF FIRST STAGE SELECTION | (FIRST) STEP OF SECOND STAGE SELECTION |
| (FIRST) GLOBAL ALIGNMENT STEP | (FIRST) LOCAL ALIGNMENT STEP |
| (FIRST) STEP OF SECOND STAGE SELECTION | (FIRST) STEP OF FIRST STAGE SELECTION |

```
         ┌──────────────────────────┐
         │   AMPLIFICATION SEQUENCE     │
         │   LENGTH-CHECKING STEP       │
         └──────────────────────────┘

┌─────────────────────────────────────────────┐
│         (FIRST) PRIMER EMPLOYMENT STEP         │
└─────────────────────────────────────────────┘
                      ↓
```

n→n+1

```
┌─────────────────────────────────────────────┐
│    n-th TARGET REGION SELECTION STEP (n≧2)    │
└─────────────────────────────────────────────┘
                      ↓
┌─────────────────────────────────────────────┐
│ n-th PRIMER CANDIDATE BASE SEQUENCE GENERATION STEP │
└─────────────────────────────────────────────┘
                      ↓
         ┌──────────────────────────┐
         │   SPECIFICITY-CHECKING STEP   │
         └──────────────────────────┘
```

| n-th LOCAL ALIGNMENT STEP | n-th GLOBAL ALIGNMENT STEP |
|---|---|
| n-th STEP OF FIRST STAGE SELECTION | n-th STEP OF SECOND STAGE SELECTION |
| n-th GLOBAL ALIGNMENT STEP | n-th LOCAL ALIGNMENT STEP |
| n-th STEP OF SECOND STAGE SELECTION | n-th STEP OF FIRST STAGE SELECTION |

```
         ┌──────────────────────────┐
         │   AMPLIFICATION SEQUENCE     │
         │   LENGTH-CHECKING STEP       │
         └──────────────────────────┘

┌─────────────────────────────────────────────┐
│          n-th PRIMER EMPLOYMENT STEP           │
└─────────────────────────────────────────────┘
```

## FIG. 2

```
alignment score = -4
[Seq ID No: 1] 5′ ～ GCTTGGCCTTGG|GAATGTGG|--------------～3′
                                   : :  | |  : : : :
[Seq ID No: 2] 3′ ～ ------------|GGTAGTAA|CCGGTATAACGG～5′
("|"=match; ":"=mismatch)
```

## FIG. 3

```
alignment score = -6
[Seq ID No: 1] 5′ ～ GCTTGG|CCTTGGGAATGTGG|-------～3′
                           | | : | : : : : : : : | :
[Seq ID No: 3] 3′ ～ ------|GGTAGGGAAGTTCG|ACGTTT～5′
("|"=match; ":"=mismatch)
```

## FIG. 4

```
alignment score = -5
[Seq ID No: 1] 5′ ～ GCTTG|GCCTTGGGAATGTGG|------～3′
                          : | : : : : : | | | : : : | :
[Seq ID No: 4] 3′ ～ -----|GGTGTATCTTTGTCT|ACGAG～5′
("|"=match; ":"=mismatch)
```

## FIG. 5

```
alignment score = -5
[Seq ID No: 1] 5′ ～ GCTTG|GCCTTGGGAATGTGG|------～3′
                          : | : | | : : : : | : | : :
[Seq ID No: 5] 3′ ～ -----|GGTAAGGGAGAGAAG|CTCCG～5′
("|"=match; ":"=mismatch)
```

## FIG. 6

```
alignment score = 0
[Seq ID No: 1] 5' ～ GCTTGG CCTTGGGAATGTGG ------- ～3'
                           | | : : | | | | : : | : : :
[Seq ID No: 6] 3' ～ ------ GGTGCCCTCTCTAA CACCAG～5'
("|"=match; ":"=mismatch)
```

## FIG. 7

```
alignment score = -4
[Seq ID No: 1] 5' ～ GCTTGGCCTT GGGAATGTGG ----------- ～3'
                              : : : : | : : : | |
[Seq ID No: 7] 3' ～ ---------- GGTCTGGGCC TGTAGAAGGA～5'
("|"=match; ":"=mismatch)
```

## FIG. 8

```
alignment score = -2
[Seq ID No: 1] 5' ～ GCTTGG CCTTGGGAATGTGG ------- ～3'
                           | | : | | : : : : | : : |
[Seq ID No: 8] 3' ～ ------ GGTACAAAGGCGTC CACCTA～5'
("|"=match; ":"=mismatch)
```

## FIG. 9

```
alignment score = -2
[Seq ID No: 2] 5' ～ GGCAATATGG CCAATGATGG ----------- ～3'
                              | | | : : : : | : :
[Seq ID No: 3] 3' ～ ---------- GGTAGGGAAG TTCGAGTTT～5'
("|"=match; ":"=mismatch)
```

## FIG. 10

```
alignment score = -3
[Seq ID No: 2] 5' ~GGCAATATGG|CCAATGATGG|————————~3'
                              | | | : : : | : : :
[Seq ID No: 4] 3' ~—————————|GGTGTATCTT|TGTCTACGAG~5'
("|"=match; ":"=mismatch)
```

## FIG. 11

```
alignment score = -2
[Seq ID No: 2] 5' ~ GGCAATATGG|CCAATGATGG|————————~3'
                               | | | : | : : : :
[Seq ID No: 5] 3' ~—————————|GGTAAGGGAG|AGAAGCTCCG~5'
("|"=match; ":"=mismatch)
```

## FIG. 12

```
alignment score = 0
[Seq ID No: 2] 5' ~ GGCAATATGG|CCAATGATGG|————————~3'
                               | | | : : | : : |
[Seq ID No: 6] 3' ~—————————|GGTGCCCTCT|CTAACACCAG~5'
("|"=match; ":"=mismatch)
```

## FIG. 13

```
alignment score = 0
[Seq ID No: 2] 5' ~ GGCAATATGG|CCAATGATGG|————————~3'
                               | | | : : : : | |
[Seq ID No: 7] 3' ~—————————|GGTCTGGGCC|TGTAGAAGGA~5'
("|"=match; ":"=mismatch)
```

## FIG. 14

```
alignment score = −4
[Seq ID No: 2] 5' ~GGCAATATGG CCAATGATGG ——————————~3'
                              | | | : : : : : : :
[Seq ID No: 8] 3' ~————————— GGTACAAAGG CGTCCACCTA~5'
(" | "=match; " : "=mismatch)
```

## FIG. 15

```
alignment score = −3
[Seq ID No: 3] 5' ~ TTT GCAGCTTGAAGGGATGG ———~3'
                        : | | : : | : | | | : : : : : |
[Seq ID No: 4] 3' ~ ——— GGTGTATCTTTGTCTAC GAG~5'
(" | "=match; " : "=mismatch)
```

## FIG. 16

```
alignment score = −9
[Seq ID No: 3] 5' ~ TTTGC AGCTTGAAGGGATGG ——————~3'
                       : : : | | : : : : : : : | : :
[Seq ID No: 5] 3' ~ ————— GGTAAGGGAGAGAAG CTCCG~5'
(" | "=match; " : "=mismatch)
```

## FIG. 17

```
alignment score = −2
[Seq ID No: 3] 5' ~ TTTG CAGCTTGAAGGGATGG —————~3'
                        | : : | : : | | : : | : : | | :
[Seq ID No: 6] 3' ~ ———— GGTGCCCTCTCTAACA CCAG~5'
(" | "=match; " : "=mismatch)
```

## FIG. 18

```
alignment score = -2
[Seq ID No: 3] 5' ～ TTTGCAGCTT GAAGGGATGG ------------ ～3'
                                  : : | | : : : : | |
[Seq ID No: 7] 3' ～ ----------- GGTCTGGGCC TGTAGAAGGA ～5'
("|"=match; ":"=mismatch)
```

## FIG. 19

```
alignment score = -5
[Seq ID No: 3] 5' ～ TTT GCAGCTTGAAGGGATGG ---～3'
                        : | | : : | | : : : | : : : : |
[Seq ID No: 8] 3' ～ --- GGTACAAAGGCGTCCAC CTA～5'
("|"=match; ":"=mismatch)
```

## FIG. 20

```
alignment score = -3
[Seq ID No: 4] 5' ～ GAGCATC TGTTTCTATGTGG --------～3'
                           : : : | | | : : | : | : :
[Seq ID No: 5] 3' ～ -------- GGTAAGGGAGAGA AGCTCCG～5'
("|"=match; ":"=mismatch)
```

## FIG. 21

```
alignment score = 0
[Seq ID No: 4] 5' ～ GAGCATCTGTTT CTATGTGG ---------------～3'
                                  : | : | : | :
[Seq ID No: 6] 3' ～ ------------ GGTGCCCT CTCTAACACCAG～5'
("|"=match; ":"=mismatch)
```

## FIG. 22

```
alignment score = -4
[Seq ID No: 4] 5' ~ GAGCATCTGTTT CTATGTGG ----------------~3'
                                     | : | : : : : :
[Seq ID No: 7] 3' ~ ------------- GGTCTGGG CCTGTAGAAGGA~5'
(”|”=match; ”:”=mismatch)
```

## FIG. 23

```
alignment score = -1
[Seq ID No: 4] 5' ~ GAGC ATCTGTTTCTATGTGG -----~3'
                        : : : | | | | | | | : : : : : | :
[Seq ID No: 8] 3' ~ ----- GGTACAAAGGCGTCCA CCTA~5'
(”|”=match; ”:”=mismatch)
```

## FIG. 24

```
alignment score = -2
[Seq ID No: 5] 5' ~ GCCTCGAA GAGAGGGAATGG ---------~3'
                               : : : : | | | | : : | :
[Seq ID No: 6] 3' ~ -------- GGTGCCCTCTCT AACACCAG~5'
(”|”=match; ”:”=mismatch)
```

## FIG. 25

```
alignment score = -4
[Seq ID No: 5] 5' ~ GCCTCGAAGA GAGGGAATGG ------------~3'
                              : : : | : : : : | |
[Seq ID No: 7] 3' ~ -------- GGTCTGGGCC TGTAGAAGGA~5'
(”|”=match; ”:”=mismatch)
```

## FIG. 26

```
alignment score = −4
[Seq ID No: 5] 5' ~ GCCTCGAAGA GAGGGAATGG ----------~3'
                               : | : | : : : : : |
[Seq ID No: 8] 3' ~ ---------- GTACAAAGGC GTCCACCTA~5'
("|"=match; ":"=mismatch)
```

## FIG. 27

```
alignment score = −2
[Seq ID No: 6] 5' ~ GACCACAA TCTCTCCCGTGG ---------~3'
                            : | : : : | | | | : : :
[Seq ID No: 7] 3' ~ -------- GGTCTGGGCCTG TAGAAGGA~5'
("|"=match; ":"=mismatch)
```

## FIG. 28

```
alignment score = −4
[Seq ID No: 6] 5' ~ GACCAC AATCTCTCCCGTGG -------~3'
                           : : : : : : | : | | | : : |
[Seq ID No: 8] 3' ~ ------ GGTACAAAGGCGTC CACCTA~5'
("|"=match; ":"=mismatch)
```

## FIG. 29

```
alignment score = 0
[Seq ID No: 7] 5' - AGGAAGATGT CCGGGTCTGG ------------~3'
                             | | : : | | : | : :
[Seq ID No: 8] 3' ~ ---------- GGTACAAAGG CGTCCACCTA~5'
("|"=match; ":"=mismatch)
```

## FIG. 30

```
alignment score = −3
5' ～ TGG ～ 3'
        : : :
3' ～ GGT ～ 5'
(":"=mismatch)
```

## FIG. 31

```
alignment score = 7
5' ～ TGCAGTCATCTTGCTCTACA————————————～3'
                 | : | | | | | | |
3' ～ ————————————AGGAGATGTCAGGGTGTTCA～5'
(" | "=match; ":"=mismatch)
```

## FIG. 32

```
alignment score = 3
5' ～ CCCAGTCAATCTAAGCCTCG————————————～3'
                 : : | : | : | : :
3' ～ ————————————GCTTGAACGGTTTCTCTAGG～5'
(" | "=match; ":"=mismatch)
```

## FIG. 33

```
alignment score = 2
5' ～ CG ～ 3'
      | |
3' ～ GC ～ 5'
(" | "=match)
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004081225 A
- WO 2008004691 A
- US 2013123120 A1
- JP 2009533030 A
- WO 2015074299 A

### Non-patent literature cited in the description

- **POE, BRIAN L. et al.** Warfarin Genotyping in a Single PCR Reaction for Microchip Electrophoresis. *Clinical Chemistry,* 2012, vol. 58 (4), 725-731
- **ALTSCHUL, S. A. et al.** Basic Local Alignment Search Tool. *Journal of Molecular Biology,* October 1990, vol. 215, 403-410
- Improved tools for biological sequence comparison. **PEARSON, W. R. et al.** Proceedings of the National Academy of Sciences of the United States of America. National Academy of Sciences, April 1988, vol. 85, 2444-2448

SEQUENCE LISTING

<110> Fuji Film Corporation

<120> A method for designing a PCR primer and a set of PCR primers

<130> W-5617PCT

<150> 2015-074299
<151> 2015-03-31

<160> 12

<170> PatentIn version 3.5

<210> 1
<211> 20
<212> DNA
<213> Artificial Sequence

<220>
<223> SNP id: rs7981616

<400> 1
gcttggcctt gggaatgtgg        20

<210> 2
<211> 20
<212> DNA
<213> Artificial Sequence

<220>
<223> SNP id: rs7981616

<400> 2
ggcaatatgg ccaatgatgg        20

<210> 3
<211> 20
<212> DNA
<213> Artificial Sequence

<220>
<223> SNP id: rs2230233

<400> 3
tttgcagctt gaagggatgg        20

<210> 4
<211> 20

<212> DNA
<213> Artificial Sequence

<220>
<223> SNP id: rs2230233

<400> 4
gagcatctgt ttctatgtgg      20

<210> 5
<211> 20
<212> DNA
<213> Artificial Sequence

<220>
<223> SNP id: rs2073370

<400> 5
gcctcgaaga gagggaatgg      20

<210> 6
<211> 20
<212> DNA
<213> Artificial Sequence

<220>
<223> SNP id: rs2073370

<400> 6
gaccacaatc tctcccgtgg      20

<210> 7
<211> 20
<212> DNA
<213> Artificial Sequence

<220>
<223> SNP id: rs2379206

<400> 7
aggaagatgt ccgggtctgg      20

<210> 8
<211> 20
<212> DNA
<213> Artificial Sequence

<220>
<223> SNP id: rs2379206

<400> 8
atccacctgc ggaaacatgg      20

<210> 9
<211> 20
<212> DNA
<213> Artificial Sequence

<220>
<223> Comparative example 1

<400> 9
tgcagtcatc ttgctctaca         20

<210> 10
<211> 20
<212> DNA
<213> Artificial Sequence

<220>
<223> Comparative example 1

<400> 10
acttgtggga ctgtagagga         20

<210> 11
<211> 20
<212> DNA
<213> Artificial Sequence

<220>
<223> Comparative example 2

<400> 11
cccagtcaat ctaagcctcg         20

<210> 12
<211> 20
<212> DNA
<213> Artificial Sequence

<220>
<223> Comparative example 2

<400> 12
ggatctcttt ggcaagttcg         20